# EUROPEAN PATENT APPLICATION

(11) **EP 4 825 837 A2**
(43) Date of publication of application: **21.10.2026**
(21) Application number: 26167056.6
(22) Date of filing: 24.03.2026
(51) Int. Cl.: C07C 67/293, C07C 29/32, C07C 45/59, C07C 67/08, C07D 307/00, C07F 9/00

(54) **PROCESSES FOR PREPARING 3-ISOPROPENYL-6-HEPTENAL COMPOUND AND 6-ISOPROPENYL-3-METHYL-3,9-DECADIENYL CARBOXYLATE COMPOUND, PROCESS FOR PREPARING INTERMEDIATE THEREOF, AND INTERMEDIATE THEREOF**

(30) Priority: 28.03.2025 JP 2025056943
(71) Applicant: SHIN-ETSU CHEMICAL CO., LTD., Tokyo 100-0005 (JP)
(72) Inventor: BABA, Akihiro, Joetsu-shi, Niigata, 942-8601 (JP); WATANABE, Takeru, Joetsu-shi, Niigata, 942-8601 (JP); FUJIMURA, Naoto, Joetsu-shi, Niigata, 942-8601 (JP)
(74) Representative: De Vries & Metman

(57) **Abstract**

The present invention relates to a process for preparing a 3-isopropenyl-6-heptenal compound (4), R³ representing a hydrogen atom or a methyl group, the process comprising at least steps of subjecting a 4-methyl-3-pentenal acetal compound (1), having a leaving group X at 5-position, R¹ and R² representing, independently of each other, a monovalent hydrocarbon group having 1 to 10 carbon atoms; or R¹ and R² being bonded to each other and representing a divalent hydrocarbon group having 2 to 10 carbon atoms, X representing an acyloxy group having 1 to 10 carbon atoms including the carbon of the carbonyl group, an alkanesulfonyloxy group having 1 to 10 carbon atoms, an arenesulfonyloxy group having 6 to 20 carbon atoms, or a halogen atom, and the wavy line representing the *E*-form, the Z-form, or a mixture thereof, to a nucleophilic substitution reaction with a nucleophilic reagent, 3-butenyl compound (2), M representing Li, MgW¹, ZnW¹, Cu, CuW¹, or CuLiW¹, and W¹ representing a halogen atom or a CH₂=C(R³)CH₂CH₂- group, to form a 3-isopropenyl-6-heptenal acetal compound (3) and subjecting the 3-isopropenyl-6-heptenal acetal compound (3) to an acid hydrolysis reaction to form the 3-isopropenyl-6-heptenal compound (4). The present invention also relates to a process for preparing the 4-methyl-3-pentenal acetal compound (1); and a process for preparing a 6-isopropenyl-3-methyl-3,9-decadienyl carboxylate compound (6) from the 4-methyl-3-pentenal acetal compound (1).

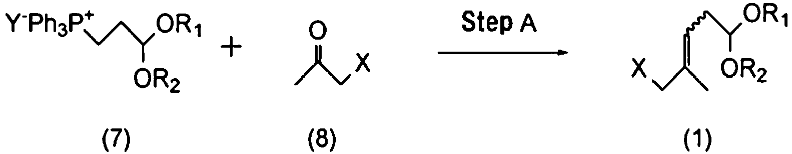

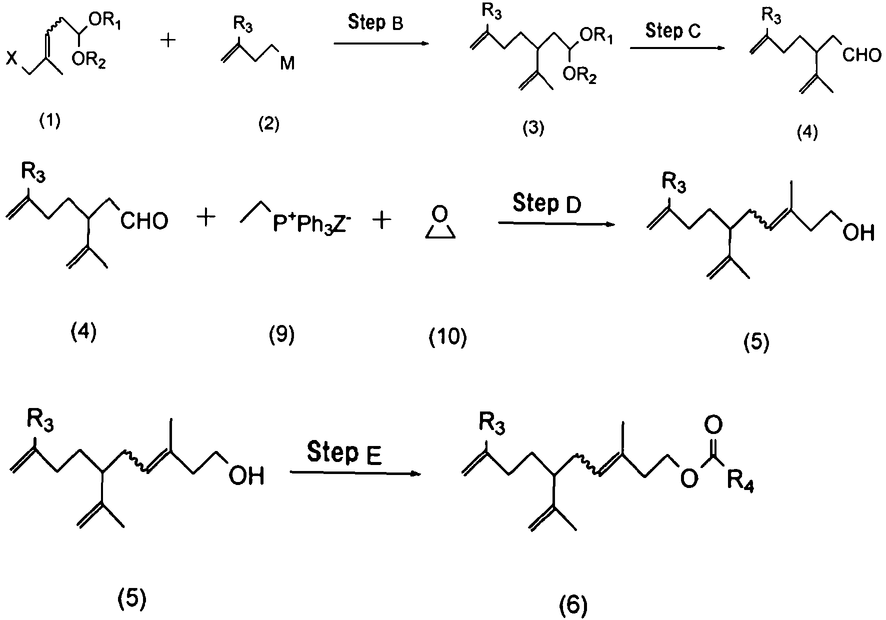

## Description

### TECHNICAL FIELD

An embodiment of the present invention relates to processes for preparing a 3-isopropenyl-6-heptenal compound and a 6-isopropenyl-3-methyl-3,9-decadienyl carboxylate compound, a process for preparing an intermediate thereof, and an intermediate thereof.

### BACKGROUND ART

Insect sex pheromones are biologically active substances which are usually borne by females to attract males, and exhibit a high attracting activity at small amounts. Sex pheromones are widely used as a means for forecasting outbreaks of pests and/or confirming their geographic spread (invasion into a specific area), and as a means for controlling pests. Widely-used means for controlling pests include mass trapping, lure-and-kill or attract-and-kill, lure-and-infect or attract-and-infect, and mating disruption.

A naturally-occurring sex pheromone may be extracted only in a trace amount from one insect, and so it is difficult to use a naturally-occurring sex pheromone for mating disruption and the like. Before practical use of a sex pheromone, it is essential to artificially prepare a sufficient amount of active agents for the sex pheromone for basic and applied research, and it is even more preferred to be able to prepare on an industrial basis.

The California red scale (scientific name: *Aonidiella aurantii*) is a pest that has spread widely throughout the world to infest citrus. (3*Z*,6*R*)-6-Isopropenyl-3-methyl-3,9-decadienyl acetate is reported as the sex pheromone of the California red scale (Non-Patent Literature 1 listed below). 6-Isopropenyl-3-methyl-3,9-decadienyl acetate includes four isomers: (3*Z*,6*R*)-6-isopropenyl-3-methyl-3,9-decadienyl acetate, (3*E*,6*R*)-6-isopropenyl-3-methyl-3,9-decadienyl acetate, (3*Z*,6*S*)-6-isopropenyl-3-methyl-3,9-decadienyl acetate, and (3*E*,6*S*)-6-isopropenyl-3-methyl-3,9-decadienyl acetate. The California red scale is attracted also by a mixture of these four isomers.

A reported process for synthesizing the aforesaid sex pheromone of the California red scale, (3*Z*,6*R*)-6-isopropenyl-3-methyl-3,9-decadienyl acetate, comprises, for example, nine steps including an oxidation reaction of (*S*)-(+)-carvone, which is a starting material, with hydrogen peroxide to obtain (*R*)-3-isopropenyl-6-heptenal; and then the (*R*)-3-isopropenyl-6-heptenal is subjected to three steps including a Wittig reaction; and the reaction product thus obtained is separated with gas-liquid chromatography (GLC) to produce (3*Z*,6*R*)-6-isopropenyl-3-methyl-3,9-decadienyl acetate (Non-Patent Literature 2 listed below).

The White peach scale (scientific name: *Pseudaulacaspis pentagona*) is a pest that has spread widely throughout the world to infest fruit trees, such as peaches, and tea. (3*Z*,6*R*)-6-Isopropenyl-3,9-dimethyl-3,9-decadienyl propionate is reported as the sex pheromone of the White peach scale (Non-Patent Literature 3 listed below). 6-Isopropenyl-3,9-dimethyl-3,9-decadienyl propionate includes four isomers: (3*Z*,6*R*)-6-isopropenyl-3,9-dimethyl-3,9-decadienyl propionate, (3*E*,6*R*)-6-isopropenyl-3,9-dimethyl-3,9-decadienyl propionate, (3*Z*,6*S*)-6-isopropenyl-3,9-dimethyl-3,9-decadienyl propionate, and (3*E*,6*S*)-6-isopropenyl-3,9-dimethyl-3,9-decadienyl propionate. The White peach scale is attracted also by a mixture of (3*Z*,6*R*)-6-isopropenyl-3,9-dimethyl-3,9-decadienyl propionate and (3*E*,6*R*)-6-isopropenyl-3,9-dimethyl-3,9-decadienyl propionate (Patent Literature 1 listed below).

A reported process for synthesizing the aforesaid sex pheromone of the White peach scale, (3*Z*,6*R*)-6-isopropenyl-3,9-dimethyl-3,9-decadienyl propionate, comprises, for example, ozonolysis of (*R*)-(+)-limonene, subjecting the product to a Wittig reaction, followed by subjecting the resulting acetal to a hydrolysis reaction to obtain (*R*)-3-isopropenyl-6-methyl-6-heptenal, then subjecting the (*R*)-3-isopropenyl-6-methyl-6-heptenal to four-carbon homologation including a Wittig reaction, followed by propionylation to obtain (3*Z*,6*R*)-6-isopropenyl-3,9-dimethyl-3,9-decadienyl propionate (Non-Patent Literature 3 listed below).

### PRIOR ART

### [Patent Literature]

[Patent Literature 1] Japanese Patent Application Laid-Open No. 2011-144114.

### [Non-Patent Literatures]

[Non-Patent Literature 1] M. J. Gieselmann et al., J. Insect. Physiol. 26, 179 (1980).
[Non-Patent Literature 2] W. Roelofs et al., J. Chem. Ecol., Vol. 4, No. 2, 211 (1978).
[Non-Patent Literature 3] R. R. Heath et al., J. Org. Chem. 45, 2910 (1980).

### OBJECT OF THE INVENTION

However, in the process of Non-Patent Literature 2, an oxidation reaction of (*S*)-(+)-carvone is carried out with hydrogen peroxide. This oxidation reaction may cause an explosion, and considering safety, is not industrially practical. Moreover, the process requires nine steps to obtain an intermediate, (*R*)-3-isopropenyl-6-heptenal, from the starting material, (*S*)-(+)-carvone, resulting in a total yield as low as 12% for the nine steps. Furthermore, the total yield to synthesize (*R*)-6-isopropenyl-3-methyl-3,9-decadienyl acetate from the starting material, (*S*)-(+)-carvone, is as low as 5.3%.

In the process of Non-Patent Literature 3, ozone, which is corrosive and highly poisonous, is used in an oxidation reaction of (*R*)-(+)-limonene. This reaction is, therefore, not industrially practical considering safety.

Thus, traditional preparation processes are considered to be extremely inadequate to prepare sufficient amounts of 6-isopropenyl-3-methyl-3,9-decadienyl acetate or 6-isopropenyl-3,9-dimethyl-3,9-decadienyl propionate on an industrial basis.

The present invention has been made in consideration of the aforesaid circumstances, and aims to provide a process for safely and efficiently preparing 6-isopropenyl-3-methyl-3,9-decadienyl carboxylate compounds, such as 6-isopropenyl-3-methyl-3,9-decadienyl acetate and 6-isopropenyl-3,9-dimethyl-3,9-decadienyl propionate, without an oxidation reaction. The present invention also aims to provide a process for preparing 3-isopropenyl-6-heptenal compounds, such as 3-isopropenyl-6-heptenal and 3-isopropenyl-6-methyl-6-heptenal, which are useful intermediates in the preparation of the aforesaid compounds. The present invention further aims to provide a 4-methyl-3-pentenal acetal compound having a leaving group X at 5-position, which is a useful common intermediate for preparing the aforesaid 6-isopropenyl-3-methyl-3,9-decadienyl carboxylate compound.

The present invention also aims to provide the aforesaid preparation processes that are useful on an industrial basis.

### SUMMARY OF THE INVENTION

As a result of intensive research to overcome the aforesaid problems of the prior art, the present inventors have found that a 4-methyl-3-pentenal acetal compound having a leaving group X at 5-position is obtained by subjecting a propyltriphenylphosphonium halide compound having an acetal at 3-position to a Wittig reaction with a 2-propanone compound having a leaving group X.

The present inventors also found that a 3-isopropenyl-6-heptenal acetal compound is obtained by subjecting a 4-methyl-3-pentenal acetal compound having a leaving group X at 5-position to a nucleophilic substitution reaction with a nucleophilic reagent, 3-butenyl compound, and that a 3-isopropenyl-6-heptenal compound is obtained by subjecting the aforesaid 3-isopropenyl-6-heptenal acetal compound to an acid hydrolysis reaction.

The present inventors also found that a 6-isopropenyl-3-methyl-3,9-decadienyl carboxylate compound is efficiently obtained using these intermediates, and thus have completed the present invention. The 6-isopropenyl-3-methyl-3,9-decadienyl carboxylate compound (6) includes the sex pheromone of the California red scale, 6-isopropenyl-3-methyl-3,9-decadienyl acetate, and the sex pheromone of the White peach scale, 6-isopropenyl-3,9-dimethyl-3,9-decadienyl propionate.

The aforesaid process for preparing the 6-isopropenyl-3-methyl-3,9-decadienyl carboxylate compound (6) was found to be suited to industrial applications. The aforesaid preparation process was found to be particularly useful in the preparation of the sex pheromone of the California red scale, 6-isopropenyl-3-methyl-3,9-decadienyl acetate, and the sex pheromone of the White peach scale, 6-isopropenyl-3,9-dimethyl-3,9-decadienyl propionate.

According to an aspect of the present invention, there is provided a process for preparing a 3-isopropenyl-6-heptenal compound of the following general formula (4): 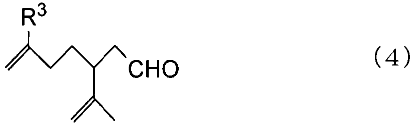 wherein R³ represents a hydrogen atom or a methyl group,
the process comprising at least steps of:
   subjecting a 4-methyl-3-pentenal acetal compound of the following general formula (1), having a leaving group X at 5-position: 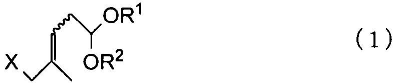
wherein R¹ and R² represent, independently of each other, a monovalent hydrocarbon group having 1 to 10 carbon atoms; or R¹ and R² are bonded to each other and represent a divalent hydrocarbon group having 2 to 10 carbon atoms, X represents an acyloxy group having 1 to 10 carbon atoms including the carbon of the carbonyl group, an alkanesulfonyloxy group having 1 to 10 carbon atoms, an arenesulfonyloxy group having 6 to 20 carbon atoms, or a halogen atom, and the wavy line represents the *E*-form, the *Z-*form, or a mixture thereof,
to a nucleophilic substitution reaction with a nucleophilic reagent, 3-butenyl compound, of the following general formula (2): 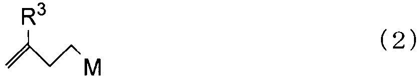
wherein R³ is as defined above, and M represents Li, MgW¹, ZnW¹, Cu, CuW¹, or CuLiW¹, and W¹ represents a halogen atom or a CH₂=C(R³)CH₂CH₂- group,
to obtain a 3-isopropenyl-6-heptenal acetal compound of the following general formula (3): 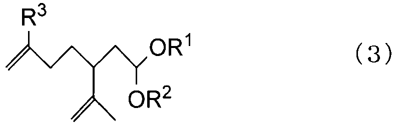
wherein R¹, R², and R³ are as defined above, and
   subjecting the 3-isopropenyl-6-heptenal acetal compound (3) thus obtained to an acid hydrolysis reaction to obtain the aforesaid 3-isopropenyl-6-heptenal compound (4).

According to another aspect of the present invention, there is provided a process for preparing a 6-isopropenyl-3-methyl-3,9-decadienyl carboxylate compound of the following general formula (6): 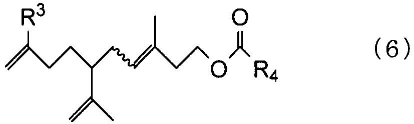 wherein R³ and the wavy line are as defined above, and R⁴ represents a hydrogen atom or a monovalent hydrocarbon group having 1 to 10 carbon atoms,
the process comprising at least steps of:
   the aforesaid process for preparing the 3-isopropenyl-6-heptenal compound (4),
   subjecting the 3-isopropenyl-6-heptenal compound (4) thus obtained to a four-carbon homologation reaction including a Wittig reaction with an ethyltriphenylphosphonium halide compound and ethylene oxide to obtain a 6-isopropenyl-3-methyl-3,9-decadien-1-ol compound of the following general formula (5): 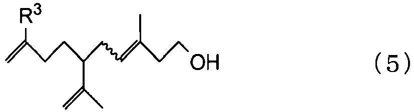
wherein R³ and the wavy line are as defined above, and
   subjecting the 6-isopropenyl-3-methyl-3,9-decadien-1-ol compound (5) thus obtained to an esterification reaction to obtain the aforesaid 6-isopropenyl-3-methyl-3,9-decadienyl carboxylate compound (6).

According to another aspect of the present invention, there is provided a process for preparing a 4-methyl-3-pentenal acetal compound of the following general formula (1), having a leaving group X at 5-position: 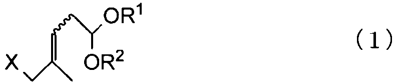 wherein R¹, R², X, and the wavy line are as defined above,
the process comprising:
   subjecting a propyltriphenylphosphonium halide compound of the following general formula (7), having an acetal at 3-position: 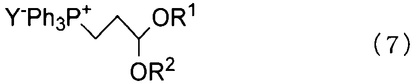
wherein R¹ and R² are as defined above, Y represents a halogen atom, and Ph represents a phenyl group,
to a Wittig reaction with a 2-propanone compound of the following general formula (8), having a leaving group X: 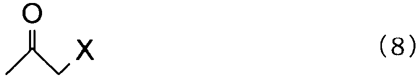
wherein X is as defined above,
to obtain the aforesaid 4-methyl-3-pentenal acetal compound (1).

According to another aspect of the present invention, there is provided a process for preparing a 3-isopropenyl-6-heptenal compound of the following general formula (4): 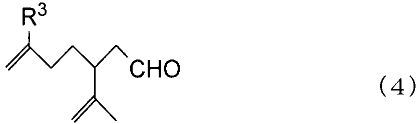 wherein R³ is as defined above,
the process comprising at least steps of:
   the aforesaid process for preparing the 4-methyl-3-pentenal acetal compound (1) having a leaving group X at 5-position,
   subjecting the aforesaid 4-methyl-3-pentenal acetal compound (1) having a leaving group X at 5-position to a nucleophilic substitution reaction with a nucleophilic reagent, 3-butenyl compound, of the following general formula (2): 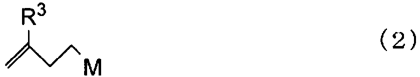
wherein R³ is as defined above, and M represents Li, MgW¹, ZnW¹, Cu, CuW¹, or CuLiW¹, and W¹ represents a halogen atom or a CH₂=C(R³)CH₂CH₂- group,
to obtain a 3-isopropenyl-6-heptenal acetal compound of the following general formula (3): 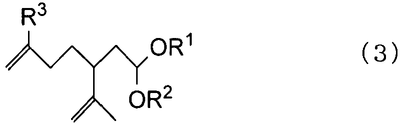
wherein R¹, R², and R³ are as defined above, and
   subjecting the 3-isopropenyl-6-heptenal acetal compound (3) thus obtained to an acid hydrolysis reaction to obtain the aforesaid 3-isopropenyl-6-heptenal compound (4).

According to another aspect of the present invention, there is provided a 4-methyl-3-pentenal acetal compound of the following general formula (1), having a leaving group X at 5-position: 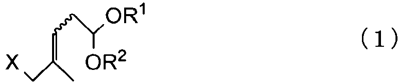 wherein R¹ and R² represent, independently of each other, a monovalent hydrocarbon group having 1 to 10 carbon atoms; or R¹ and R² are bonded to each other and represent a divalent hydrocarbon group having 2 to 10 carbon atoms, X represents an acyloxy group having 1 to 10 carbon atoms including the carbon of the carbonyl group, an alkanesulfonyloxy group having 1 to 10 carbon atoms, an arenesulfonyloxy group having 6 to 20 carbon atoms, or a halogen atom, and the wavy line represents the *E*-form, the *Z-*form, or a mixture thereof.

According to the present invention, there is provided a process of efficiently preparing a 6-isopropenyl-3-methyl-3,9-decadienyl carboxylate compound, such as 6-isopropenyl-3-methyl-3,9-decadienyl acetate and 6-isopropenyl-3,9-dimethyl-3,9-decadienyl propionate, without requiring an oxidation reaction or a reduction reaction. The present invention also may provide a process for preparing a 6-isopropenyl-3-methyl-3,9-decadienyl carboxylate compound via useful intermediates, 3-isopropenyl-6-heptenal and 3-isopropenyl-6-methyl-6-heptenal. The present invention also may provide a process for preparing a useful common intermediate, 4-methyl-3-pentenal acetal compound having a leaving group X at 5-position, for preparing the aforesaid 6-isopropenyl-3-methyl-3,9-decadienyl carboxylate compound.

The aforesaid preparation processes do not require industrially impractical oxidation reactions or reduction reactions, and are therefore useful on an industrial basis in view of safety, economy, and/or environmental impact.

### DETAILED DESCRIPTION OF THE INVENTION

Embodiments of the present invention will be explained in detail below. It should be understood that the present invention is not limited to or by the following embodiments.

Structures of intermediates, reagents, and target compounds represented by chemical formulae in the present specification may sometimes comprise stereoisomers, such as enantiomers or diastereomers. Unless otherwise stated, the chemical formulae shall be interpreted to represent all of these isomers. The isomers may be used alone or in combination at any ratio. A 50:50 mixture of two enantiomers is a racemic mixture, and a mixture containing two enantiomers at a non-equivalent ratio is a scalemic mixture. Either a racemic mixture or a scalemic mixture may be used.

In the formulas, the wavy line represents a bond without a specific geometrical isomerism, and represents one or both of a (*E*)-isomer and a (*Z*)-isomer.

Unless otherwise noted, stereochemical notation in the present specification follows the recommendations of the International Union of Pure and Applied Chemistry: IUPAC Recommendations 2006; Pure Appl. Chem., 78, 1897, (2006).

The present inventors have contemplated a plan for the synthesis of a 6-isopropenyl-3-methyl-3,9-decadienyl carboxylate compound (6) of the following general formula (6), as follows:

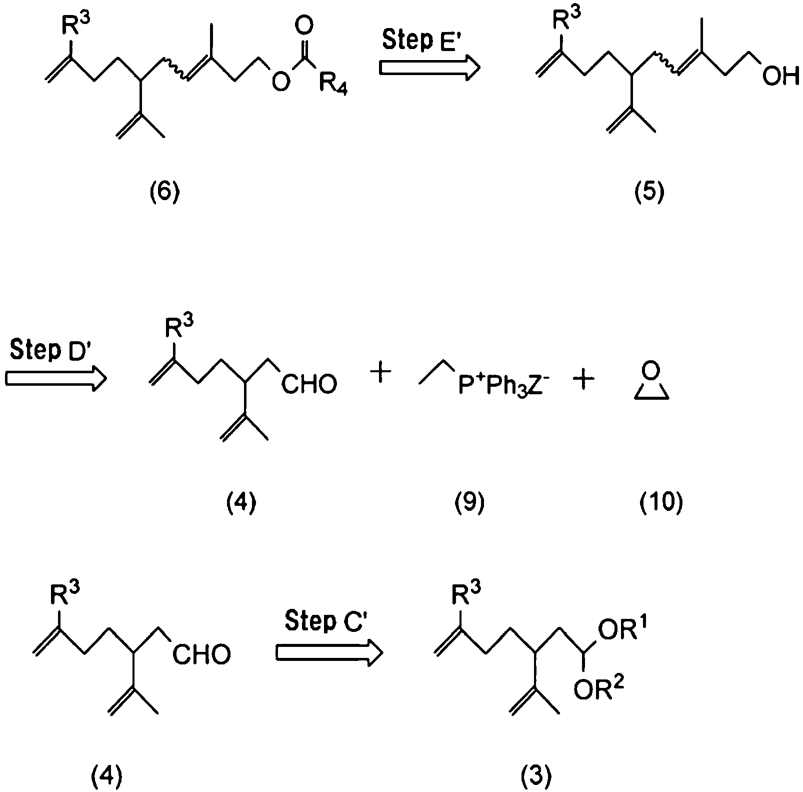

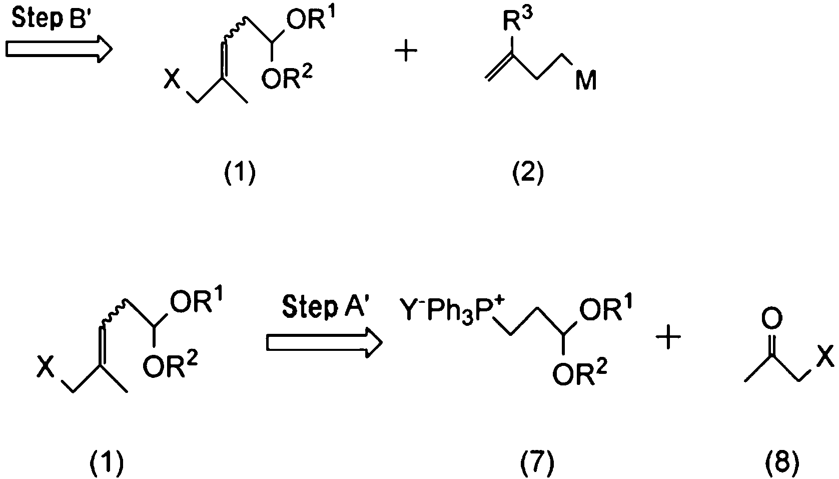

In the formula, the open arrow represents a transform in retrosynthetic analysis. Y and Z represent, independently of each other, a halogen atom. R¹ and R² represent, independently of each other, a monovalent hydrocarbon group having 1 to 10 carbon atoms; or R¹ and R² are bonded to each other and represent a divalent hydrocarbon group having 2 to 10 carbon atoms. R³ represents a hydrogen atom or a methyl group. X represents an acyloxy group having 1 to 10 carbon atoms including the carbon of the carbonyl group, an alkanesulfonyloxy group having 1 to 10 carbon atoms, an arenesulfonyloxy group having 6 to 20 carbon atoms, or a halogen atom. The wavy line represents the *E*-form, the *Z*-form, or a mixture thereof.

In the aforesaid synthesis plan, a compound of general formula (6) when R³ is a hydrogen atom and R⁴ is a methyl group, that is, 6-isopropenyl-3-methyl-3,9-decadienyl acetate, is the sex pheromone of the California red scale, and a compound of general formula (6) when R³ is a methyl group and R⁴ is an ethyl group, that is, 6-isopropenyl-3,9-dimethyl-3,9-decadienyl propionate, is the sex pheromone of the White peach scale.

The present inventors have now found a 4-methyl-3-pentenal acetal compound (1) having a leaving group X at 5-position as a key synthetic intermediate.

### Step E'

The target compound of the present invention, 6-isopropenyl-3-methyl-3,9-decadienyl carboxylate compound (6), is considered to be synthesized by subjecting a 6-isopropenyl-3-methyl-3,9-decadien-1-ol compound (5) to an esterification reaction with an esterifier.

The aforesaid formula (6) represents one or a mixture of two or more of a (3*Z*,6*R*)-6-isopropenyl-3-methyl-3,9-decadienyl carboxylate compound of the following formula (6a), a (3*E*,6*R*)-6-isopropenyl-3-methyl-3,9-decadienyl carboxylate compound of the following formula (6b), a (3*Z*,6*S*)-6-isopropenyl-3-methyl-3,9-decadienyl carboxylate compound of the following formula (6c), and a (3*E*,6*S*)-6-isopropenyl-3-methyl-3,9-decadienyl carboxylate compound of the following formula (6d):

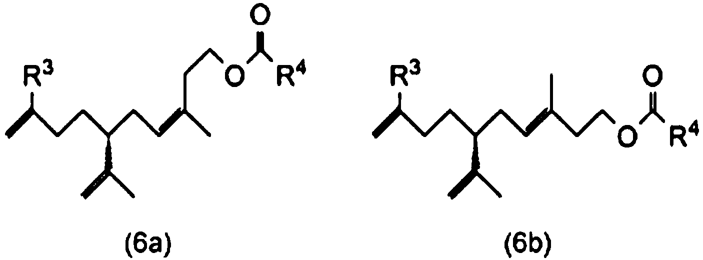

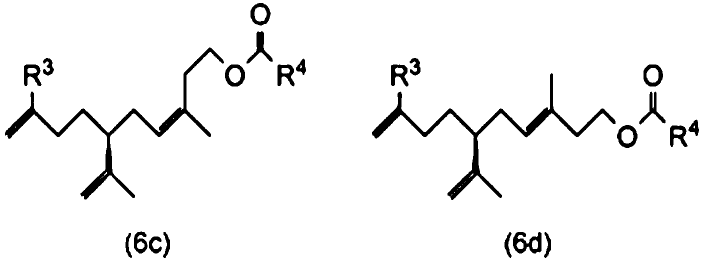

The aforesaid formula (5) represents one or a mixture of two or more of a (3*Z*,6*R*)-6-isopropenyl-3-methyl-3,9-decadien-1-ol compound of the following formula (5a), a (3*E*,6*R*)-6-isopropenyl-3-methyl-3,9-decadien-1-ol compound of the following formula (5b), a (3*Z*,6*S*)-6-isopropenyl-3-methyl-3,9-decadien-1-ol compound of the following formula (5c), and a (3*E*,6*S*)-6-isopropenyl-3-methyl-3,9-decadien-1-ol compound of the following formula (5d):

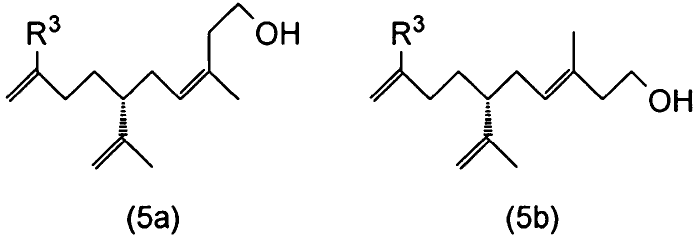

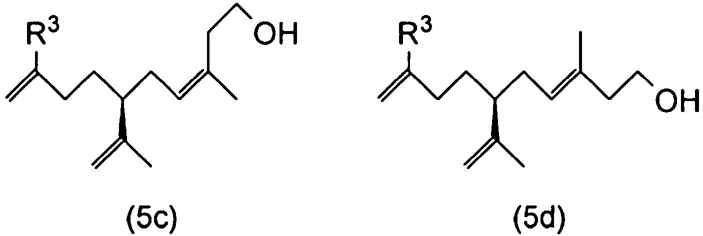

### Step D'

The target compound, 6-isopropenyl-3-methyl-3,9-decadien-1-ol compound (5), is considered to be synthesized by subjecting a 3-isopropenyl-6-heptenal compound (4) to a four-carbon homologation reaction including a Wittig reaction with an ethyltriphenylphosphonium halide compound (9) and ethylene oxide (10).

The aforesaid formula (4) represents one or both of a (*R*)-3-isopropenyl-6-heptenal compound of the following formula (4a) and a (*S*)-3-isopropenyl-6-heptenal compound of the following formula (4b):

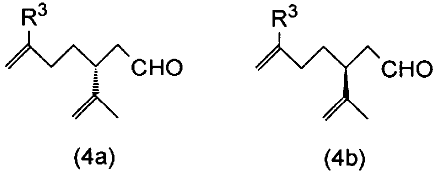

### Step C'

The target compound of this step, 3-isopropenyl-6-heptenal compound (4), is considered to be synthesized by subjecting a 3-isopropenyl-6-heptenal acetal compound (3) to an acid hydrolysis reaction. The 3-isopropenyl-6-heptenal compound (4) is considered to be an important intermediate in the preparation of a 6-isopropenyl-3-methyl-3,9-decadienyl carboxylate compound (6).

The aforesaid formula (3) represents one or both of a (*R*)-3-isopropenyl-6-heptenal acetal compound of the following formula (3a) and a (*S*)-3-isopropenyl-6-heptenal acetal compound of the following formula (3b):

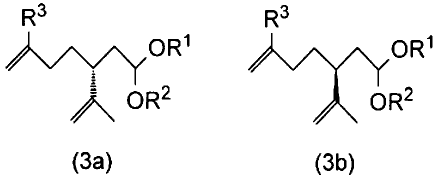

### Step B'

The target compound, 3-isopropenyl-6-heptenal acetal compound (3), is considered to be synthesized by subjecting a 4-methyl-3-pentenal acetal compound (1) having a leaving group X at 5-position to a nucleophilic substitution reaction with a nucleophilic reagent, 3-butenyl compound (2). Specifically, in the nucleophilic substitution reaction between the 4-methyl-3-pentenal acetal compound (1) having a leaving group X at 5-position and the nucleophilic reagent, 3-butenyl compound (2), if the carbon atom of position 3 of the 4-methyl-3-pentenal acetal compound (1) having a leaving group X at 5-position may be selectively attacked (a so-called S_{N}2' mechanism), a nucleophilic substitution reaction with allylic rearrangement may occur, and a 3-isopropenyl-6-heptenal acetal compound (3) may be synthesized.

The aforesaid formula (1) represents one or both of a (*Z*)-4-methyl-3-pentenal acetal compound having a leaving group X at 5-position of the following formula (1a) and a (*E*)-4-methyl-3-pentenal acetal compound of the following formula (1b):

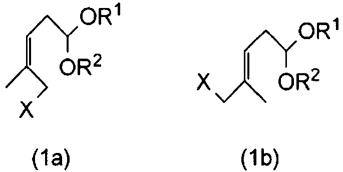

### Step A'

The target compound of the present invention, 4-methyl-3-pentenal acetal compound (1) having a leaving group X at 5-position, may be synthesized by a Wittig reaction between a propyltriphenylphosphonium halide compound (7) having an acetal at 3-position and a 2-propanone compound (8) having a leaving group X.

The aforesaid process for synthesizing does not require an oxidation reaction or a reduction reaction, and is therefore considered to be able to safely and efficiently prepare a 6-isopropenyl-3-methyl-3,9-decadienyl carboxylate compound (6).

Furthermore, the aforesaid process for synthesizing does not require industrially impractical oxidation reactions and reduction reactions, and is therefore considered to be able to prepare a 6-isopropenyl-3-methyl-3,9-decadienyl carboxylate compound (6) on an industrial basis.

If a 6-isopropenyl-3-methyl-3,9-decadienyl carboxylate compound (6) can be prepared by the aforesaid synthesizing process, the 4-methyl-3-pentenal acetal compound (1) having a leaving group X at 5-position can be expected to have an advantage of being a common intermediate usable in both preparation of the sex pheromone of the California red scale and preparation of the sex pheromone of the White peach scale.

In consideration of the reaction scheme of the retrosynthetic analysis mentioned above, an embodiment of the present invention may be depicted by the following chemical reaction scheme.

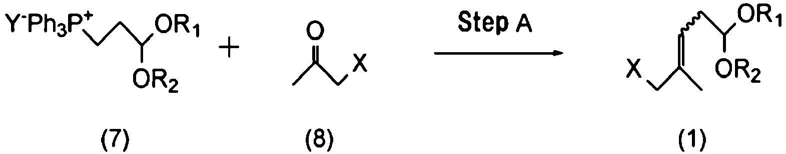

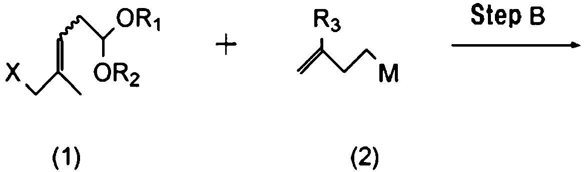

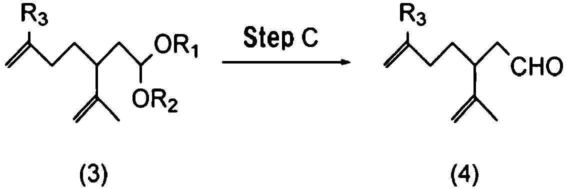

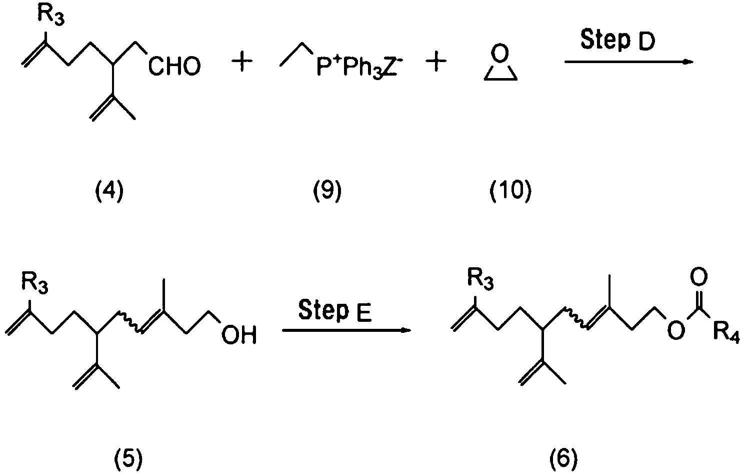

As a result of intensive research based on the aforesaid investigation, the present inventors successfully realized the desired reaction, and efficiently synthesized a 6-isopropenyl-3-methyl-3,9-decadienyl carboxylate compound (6).

Specifically, a 3-isopropenyl-6-heptenal compound (4) may be synthesized without using an oxidation reaction or a reduction reaction by subjecting a propyltriphenylphosphonium halide compound (7) having an acetal at 3-position to a Wittig reaction with a 2-propanone compound (8) having a leaving group X to obtain a 4-methyl-3-pentenal acetal compound (1) having a leaving group X at 5-position, subjecting the aforesaid 4-methyl-3-pentenal acetal compound (1) having a leaving group X at 5-position to a nucleophilic substitution reaction with a nucleophilic reagent, 3-butenyl compound (2), and then subjecting the resulting acetal to an acid hydrolysis reaction. As a result, the 3-isopropenyl-6-heptenal compound (4) may be used as an intermediate to easily produce a 6-isopropenyl-3-methyl-3,9-decadienyl carboxylate compound (6) from the 3-isopropenyl-6-heptenal compound (4).

The aforesaid Steps A to E will be explained in detail below as an embodiment of the present invention. First, Step B of synthesizing a 3-isopropenyl-6-heptenal acetal compound (3), then Step C of synthesizing the target compound of the present invention, 3-isopropenyl-6-heptenal compound (4), followed by Step D of synthesizing a 6-isopropenyl-3-methyl-3,9-decadien-1-ol compound (5), and then Step E of synthesizing the target compound of the present invention, 6-isopropenyl-3-methyl-3,9-decadienyl carboxylate compound (6), will be explained in this order. Step A of synthesizing the target compound of the present invention, 4-methyl-3-pentenal acetal compound (1) having a leaving group X at 5-position, will then be explained in detail below.

An embodiment of the present invention will be explained in detail below.

### [1] Step B

Step B of synthesizing a 3-isopropenyl-6-heptenal acetal compound (3) will be explained in detail below. As shown in the following chemical reaction formula, a 3-isopropenyl-6-heptenal acetal compound (3) is obtained by subjecting a 4-methyl-3-pentenal acetal compound (1) having a leaving group X at 5-position obtained by Step A, that will be explained below, to a nucleophilic substitution reaction with a nucleophilic reagent, 3-butenyl compound (2).

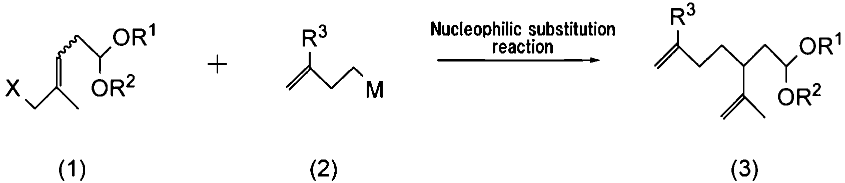

First, a 4-methyl-3-pentenal acetal compound having a leaving group X at 5-position of the following general formula (1) will be explained.

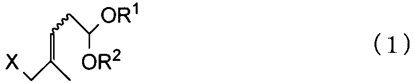

A 4-methyl-3-pentenal acetal compound (1) having a leaving group X at 5-position may include a (*Z*)-4-methyl-3-pentenal acetal compound having a leaving group X at 5-position of the following formula (1a), and a (E)-4-methyl-3-pentenal acetal compound of the following formula (1b). These isomers may be alone or in combination thereof because these isomers converge to isopropenyl groups with rearrangement of the double bond during the nucleophilic substitution reaction.

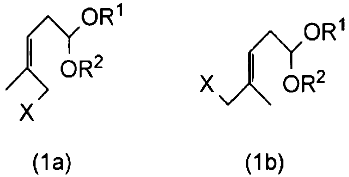

R¹ and R² represent, independently of each other, a monovalent hydrocarbon group having 1 to 10 carbon atoms, preferably 1 to 4 carbon atoms, and more preferably 1 to 3 carbon atoms; or R¹ and R² are bonded to each other and represent a divalent hydrocarbon group having 2 to 10 carbon atoms, preferably 2 to 6 carbon atoms, more preferably 2 to 4 carbon atoms, and even more preferably 2 to 3 carbon atoms.

Examples of the monovalent hydrocarbon group include linear saturated hydrocarbon groups such as a methyl group, an ethyl group, an n-propyl group, an n-butyl group, and an n-pentyl group; branched saturated hydrocarbon groups such as an isopropyl group; linear unsaturated hydrocarbon groups such as a 2-propenyl group and a 2-propynyl group; branched unsaturated hydrocarbon groups such as a 2-methyl-2-propenyl group; cyclic hydrocarbon groups such as a cyclopropyl group, a 2-methylcyclopropyl group, a cyclobutyl group, and a cyclopentyl group; and isomers thereof. A part of the hydrogen atoms in the hydrocarbon groups may be substituted with a methyl group or an ethyl group. The hydrocarbon group is preferably a chain-like saturated hydrocarbon group. By using said chain-like saturated hydrocarbon group, a preferred reactivity and ease of purification may be ensured. The hydrocarbon group is more preferably a methyl group, an ethyl group, and an n-propyl group. By using said methyl group, ethyl group, and n-propyl group, a more preferred reactivity and ease of purification may be ensured.

Examples of the divalent hydrocarbon group include linear saturated hydrocarbon groups such as an ethylene group, a 1,3-propylene group, and a 1,4-butylene group; branched saturated hydrocarbon groups such as a 1,2-propylene group, a 2,2-dimethyl-1,3-propylene group, a 1,2-butylene group, a 1,3-butylene group, a 2,3-butylene group, and a 2,3-dimethyl-2,3-butylene group; linear unsaturated hydrocarbon groups such as a (*Z*)-2-butene-1,4-diyl group; branched unsaturated hydrocarbon groups such as a 1-vinylethylene group, and a 2-methylene-1,3-propylene group; cyclic hydrocarbon groups such as a 1,2-cyclopropylene group, a 1,2-cyclobutylene group, a 1,2-cyclopentylene group, a 1,2-cyclohexylene group, and a 1,2-phenylene group; and isomers thereof. A part of the hydrogen atoms in the hydrocarbon groups may be substituted with a methyl group or an ethyl group. The hydrocarbon group is preferably a linear saturated hydrocarbon group. By using said linear saturated hydrocarbon group, a preferred reactivity of deprotection, ease of purification, and/or availability may be ensured. The hydrocarbon group is more preferably an ethylene group and a 1,3-propylene group. By using said ethylene group and 1,3-propylene group, a more preferred reactivity of deprotection, ease of purification, and/or availability may be ensured.

X represents an acyloxy group including the carbon of the carbonyl group, and having 1 to 10 carbon atoms, preferably 1 to 8 carbon atoms, and more preferably 1 to 6 carbon atoms; an alkanesulfonyloxy group having 1 to 10 carbon atoms, and preferably 1 to 3 carbon atoms; an arenesulfonyloxy group having 6 to 20 carbon atoms, and preferably 6 to 10 carbon atoms; and a halogen atom. X may be appropriately selected from these in view of the reactivity, reaction selectivity, availability of raw materials, ease of synthesis, storage stability, and/or price.

Examples of the acyloxy group having 1 to 10 carbon atoms including the carbon of the carbonyl group include linear aliphatic acyloxy groups such as a formyloxy group, an acetoxy group, a propanoyloxy group, a butanoyloxy group, a pentanoyloxy group, a hexanoyloxy group, a heptanoyloxy group, an octanoyloxy group, a nonanoyloxy group, a decanoyloxy group, and a crotonyloxy group; branched aliphatic acyloxy groups such as a 2-methylpropanoyloxy group, a pivaloyloxy group, a 2-methylbutanoyloxy group, a 3-methyl-2-butenoyloxy group, and a 3-methyl-3-butenoyloxy group; halogenated acyloxy groups such as a trichloroacetoxy group and a trifluoroacetoxy group; aromatic acyloxy groups such as a benzoyloxy group; and isomers thereof. A part of the hydrogen atoms of the acyloxy group may be substituted with a methyl group, an ethyl group, or a halogen atom. Examples of the halogen atom include a chlorine atom, a bromine atom, and an iodine atom.

The acyloxy group is preferably an acetoxy group, a propanoyloxy group, a pivaloyloxy group, a 2-methylpropanoyloxy group, and a benzoyloxy group. By using said acetoxy group, propanoyloxy group, pivaloyloxy group, 2-methylpropanoyloxy group, and benzoyloxy group, a preferred availability may be ensured.

Examples of the alkanesulfonyloxy group having 1 to 10 carbon atoms include a methanesulfonyloxy group, an ethanesulfonyloxy group, a 1-butanesulfonyloxy group, a 1-pentanesulfonyloxy group, a 1-hexanesulfonyloxy group, a 1-heptanesulfonyloxy group, a 1-octanesulfonyloxy group, a 1-nonanesulfonyloxy group, a 1-decanesulfonyloxy group, an allylsulfonyloxy group, a 10-camphorsulfonyloxy group, a trifluoromethanesulfonyloxy group, an α-benzylsulfonyloxy group, and isomers thereof. A part of the hydrogen atoms of the alkanesulfonyloxy group may be substituted with a methyl group, an ethyl group, or a halogen atom. Examples of the halogen atom include a chlorine atom, a bromine atom, and an iodine atom.

The alkanesulfonyloxy group is preferably a methanesulfonyloxy group and an ethanesulfonyloxy group. By using said methanesulfonyloxy group and ethanesulfonyloxy group, a preferred availability may be ensured.

Examples of the arenesulfonyloxy group having 6 to 20 carbon atoms include a benzenesulfonyloxy group, a 4-chlorobenzenesulfonyloxy group, a 4-methoxybenzenesulfonyloxy group, a 2-nitrobenzenesulfonyloxy group, a 2,4,6-trimethylbenzenesulfonyloxy group, a *p*-toluenesulfonyloxy group, a 1-naphthalenesulfonyloxy group, a 2-naphthalenesulfonyloxy group, and isomers thereof. A part of the hydrogen atoms of the arenesulfonyloxy group may be substituted with a methyl group, an ethyl group, or a halogen atom. Examples of the halogen atom include a chlorine atom, a bromine atom, and an iodine atom.

The arenesulfonyloxy group is preferably a benzenesulfonyloxy group and a *p-*toluenesulfonyloxy group. By using said benzenesulfonyloxy group and *p-*toluenesulfonyloxy group, a preferred availability may be ensured.

Examples of the halogen atom of X include a fluorine atom, a chlorine atom, a bromine atom, and an iodine atom.

The halogen atom is preferably a chlorine atom and a bromine atom. By using said chlorine atom and bromine atom, a preferred availability may be ensured.

Specific examples of the 4-methyl-3-pentenal acetal compound (1) having a leaving group X at 5-position include 4-methyl-3-pentenal acetal compounds having an acyloxy group at 5-position such as 5-acetoxy-4-methyl-3-pentenal dimethyl acetal, 5-acetoxy-4-methyl-3-pentenal diethyl acetal, 5-acetoxy-4-methyl-3-pentenal ethylene acetal, and 5-isopropyloxy-4-methyl-3-pentenal diethyl acetal; 4-methyl-3-pentenal acetal compounds having an alkanesulfonyloxy group at 5-position such as 5-methanesulfonyloxy-4-methyl-3-pentenal diethyl acetal; 4-methyl-3-pentenal acetal compounds having an arenesulfonyloxy group at 5-position such as 4-methyl-5-p-toluenesulfonyloxy-3-pentenal diethyl acetal; and 4-methyl-3-pentenal acetal compounds having a halogen at 5-position such as 5-chloro-4-methyl-3-pentenal diethyl acetal.

Next, a nucleophilic reagent, 3-butenyl compound, of the following general formula (2) will be explained.

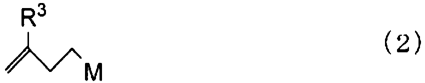

In the nucleophilic reagent, 3-butenyl compound (2), R³ represents a hydrogen atom or a methyl group.

In the nucleophilic reagent, 3-butenyl compound (2), M represents Li, MgW¹, ZnW¹, Cu, CuW¹, or CuLiW¹, and W¹ represents a halogen atom or a CH₂=C(R³)CH₂CH₂- group. Examples of the halogen atom of W¹ include a fluorine atom, a chlorine atom, a bromine atom, and an iodine atom.

The nucleophilic reagent, 3-butenyl compound (2), is preferably organolithium reagents such as a 3-butenyllithium compound; and organomagnesium reagents such as a 3-butenylmagnesium halide compound (a Grignard reagent). By using said organolithium reagents such as a 3-butenyllithium compound; and organomagnesium reagents such as a 3-butenylmagnesium halide compound, a preferred reactivity, selectivity, and/or ease of preparation may be ensured. The nucleophilic reagent, 3-butenyl compound (2), is particularly preferably a 3-butenylmagnesium halide compound (a Grignard reagent). By using said 3-butenylmagnesium halide compound, a particularly preferred reactivity, selectivity, and/or ease of preparation may be ensured.

Specific examples of the halide of the 3-butenylmagnesium halide compound include a 3-butenylmagnesium chloride compound, a 3-butenylmagnesium bromide compound, and a 3-butenylmagnesium iodide compound.

The nucleophilic reagent, 3-butenyl compound (2), is prepared by a conventional process from, for example, its corresponding halide, 3-butenyl halide compound. Examples of the 3-butenyl halide compound include a 3-butenyl chloride compound, a 3-butenyl bromide compound, and a 3-butenyl iodide compound. The nucleophilic reagent, 3-butenyl compound (2), is preferably a 3-butenyl chloride compound and a 3-butenyl bromide compound. By using said 3-butenyl chloride compound and 3-butenyl bromide compound, a preferred ease of preparation and/or stability of compounds may be ensured.

The nucleophilic reagent, 3-butenyl compound (2), may be prepared by subjecting an organolithium reagent or an organomagnesium reagent to a metal exchange reaction with a stoichiometric amount (1 mol or more) of a transition metal compound, or may be formed in-situ by reacting an organolithium reagent or a Grignard reagent with a catalytic amount of a transition metal compound. Preferably, a nucleophilic reagent, 3-butenyl compound (2), is formed in-situ by reacting an organolithium reagent or a Grignard reagent with a catalytic amount of a transition metal compound.

Examples of the transition metal compound include transition metal compounds containing copper, iron, nickel, palladium, zinc, titanium, or silver. Preferred are cuprous halides such as copper(I) chloride, copper(I) bromide, and copper(I) iodide; cupric halides such as copper(II) chloride, copper(II) bromide, and copper(II) iodide; copper cyanides such as copper(I) cyanide and copper(II) cyanide; copper oxides such as copper(I) oxide and copper(II) oxide; and copper compounds such as dilithium tetrachlorocuprate (Li₂CuCl₄). Cuprous halides are particularly preferred. By using said cuprous halides, a particularly preferred reactivity may be ensured.

The amount of the transition metal compound used may be a catalytic amount (0.0001 mol to 0.999 mol), a stoichiometric amount (1 mol), or an excessive amount (more than 1 mol up to 100 mol). The amount of the transition metal compound used is preferably 0.0001 mol to 10 mol.

Here, in the 4-methyl-3-pentenal acetal compound (1) having a leaving group X at 5-position, the leaving group is at an allyl position. Therefore, the 4-methyl-3-pentenal acetal compound (1) having a leaving group at 5-position may be attacked by the nucleophilic reagent, 3-butenyl compound (2), at the carbon atom at 3-position and at the carbon atom at 5-position to which the leaving group X is bonded.

When the nucleophilic reagent, 3-butenyl compound (2), attacks the carbon atom at 3-position of the 4-methyl-3-pentenal acetal compound (1) having a leaving group X at 5-position (a so-called S_{N}2' mechanism), a nucleophilic substitution reaction with allylic rearrangement occurs, and a 3-isopropenyl-6-heptenal acetal compound (3) is produced.

Meanwhile, when the nucleophilic reagent, 3-butenyl compound (2), attacks the carbon atom at 5-position of the 4-methyl-3-pentenal acetal compound (1) having a leaving group X at 5-position (a so-called S_{N}2 mechanism), a 4-methyl-3,8-nonadienal acetal compound of the following general formula (3') is produced.

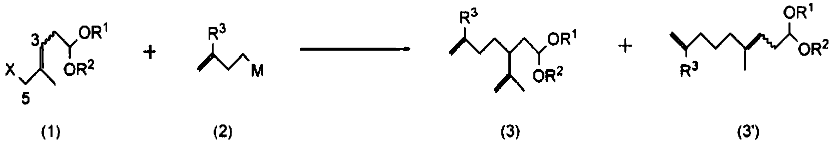

That is, the production of the 3-isopropenyl-6-heptenal acetal compound (3) may compete with the 4-methyl-3,8-nonadienal acetal compound (3') in Step B. Therefore, optimal conditions may be adopted, among the nucleophilic substitution reaction conditions that will be explained below, to reduce the by-production of the 4-methyl-3,8-nonadienal acetal compound (3') and to produce the 3-isopropenyl-6-heptenal acetal compound (3) with good yield.

The 3-isopropenyl-6-heptenal acetal compound (3) may include a (R)-3-isopropenyl-6-heptenal acetal compound of the following formula (3a) and a (S)-3-isopropenyl-6-heptenal acetal compound of the following formula (3b). These isomers may be used alone or in combination thereof, and preferably include, for example, the aforesaid compound (3a), which has the same skeleton as the natural sex pheromones of females of the California red scale and the White peach scale.

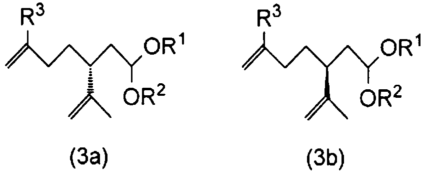

In the 3-isopropenyl-6-heptenal acetal compound (3), R¹ and R² are as defined for general formula (1). R³ is as defined for general formula (2).

The 4-methyl-3,8-nonadienal acetal compound (3') by-produced in the nucleophilic substitution reaction may include a (*Z*)-4-methyl-3,8-nonadienal acetal compound of the following formula (3'a), a (*E*)-4-methyl-3,8-nonadienal acetal compound of the following formula (3'b), or a mixture thereof.

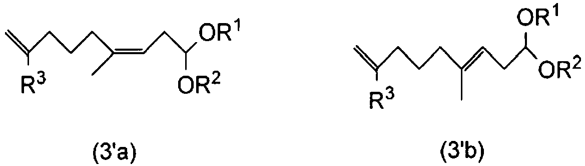

Preferably, the 4-methyl-3-pentenal acetal compound (1) having a leaving group X at 5-position is subjected to a nucleophilic substitution reaction with the nucleophilic reagent, 3-butenyl compound (2), in the presence of a co-catalyst such as phosphorus compounds such as trialkyl phosphites such as triethyl phosphite; and triarylphosphines such as triphenylphosphine, in an amount of 0.001 mol to 1,000 mol, per mol of a transition metal compound. By using said co-catalyst and said amount, a preferred solubility of the transition metal compound in a solvent may be ensured.

The nucleophilic substitution reaction also may be carried out in the presence of a reaction catalyst such as lithium salts such as lithium chloride, lithium bromide, and lithium iodide, in an amount of 0.001 mol to 1,000 mol, per mol of the 4-methyl-3-pentenal acetal compound (1) having a leaving group X at 5-position.

In the nucleophilic substitution reaction, a combination of a cuprous halide and a lithium salt is particularly preferred. By using said combination of a cuprous halide and a lithium salt, a particularly preferred reactivity including a product ratio of a target compound to a by-product may be ensured.

The amount of the nucleophilic reagent, 3-butenyl compound (2), used may be arbitrarily set in view of the type of reagent, reaction conditions, a reaction yield, economy such as prices of intermediates, and/or ease of isolation or purification of the target compound from a reaction product. The amount of the nucleophilic reagent, 3-butenyl compound (2), used is preferably 0.2 mol to 100 mol, more preferably 0.5 mol to 20 mol, and even more preferably 0.8 mol to 5 mol, per mol of the 4-methyl-3-pentenal acetal compound (1) having a leaving group X at 5-position.

Nucleophilic substitution reaction is carried out in the presence of a solvent and, if necessary, under heating or cooling.

Preferably, the solvent used in the nucleophilic substitution reaction includes ethers such as diethyl ether, di-n-butyl ether, *t*-butyl methyl ether, cyclopentyl methyl ether, tetrahydrofuran, 2-methyltetrahydrofuran, and 1,4-dioxane. Hydrocarbons such as hexane, heptane, benzene, toluene, xylene, and cumene; and aprotic polar solvents such as *N,N*-dimethylformamide (DMF), *N,N*-dimethylacetamide, *N,N-*dimethylpropionamide, 1,3-dimethyl-2-imidazolidinone (DMI), dimethyl sulfoxide (DMSO), and hexamethylphosphoric triamide (HMPA) may be mixed with the solvent. The solvent may be a commercially available one.

The amount of the solvent used in the nucleophilic substitution reaction is not particularly limited, and is preferably 10 g to 1,000,000 g, more preferably 100 g to 100,000 g, and even more preferably 150 g to 10,000 g, per mol of the nucleophilic reagent, 3-butenyl compound (2).

The reaction temperature of the nucleophilic substitution reaction is preferably from -78°C to the boiling point of the solvent, more preferably -78°C to 100°C, and even more preferred -78°C to 50°C.

The reaction time of the nucleophilic substitution reaction may be arbitrarily set, and may be optimized by monitoring progress of the reaction with gas chromatography (GC) and/or thin layer chromatography (TLC). The reaction time is typically and preferably 5 minutes to 240 hours.

When the 3-isopropenyl-6-heptenal acetal compound (3) obtained in the nucleophilic substitution reaction has a sufficient purity, the crude product may be used as such in a subsequent step. Alternatively, the crude product may be suitably purified by any purification method used in usual organic synthesis, such as distillation or various chromatography.

### [2] Step C

Step C of synthesizing a 3-isopropenyl-6-heptenal compound (4) will be explained in detail below. A 3-isopropenyl-6-heptenal compound (4) is obtained by subjecting the 3-isopropenyl-6-heptenal acetal compound (3) obtained in Step B to an acid hydrolysis reaction, as shown in the following chemical reaction formula:

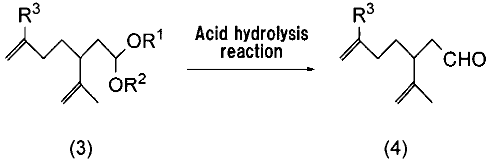

A 3-isopropenyl-6-heptenal compound of the following general formula (4) will be described below.

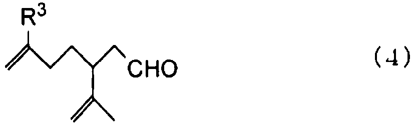

The 3-isopropenyl-6-heptenal compound (4) may include a (R)-3-isopropenyl-6-heptenal compound of the following formula (4a) and a (S)-3-isopropenyl-6-heptenal compound of the following formula (4b). These isomers may be alone or in combination thereof, and preferably include, for example, the aforesaid compound (4a), which has the same skeleton as the natural sex pheromone of the females of the California red scale and the White peach scale. 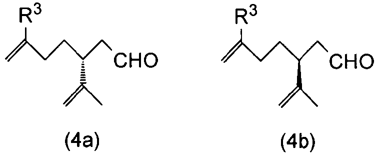 R³ is as defined for general formula (2) above.

Specific examples of the 3-isopropenyl-6-heptenal compound (4) include 3-isopropenyl-6-heptenal (left side) and 3-isopropenyl-6-methyl-6-heptenal (right side) shown below.

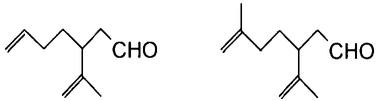

In the deprotection reaction, an isomer (hereinafter, also referred to as "the isomer (4')") may be by-produced, wherein a double bond of an isopropenyl group of a 3-isopropenyl-6-heptenal acetal compound (3) of the following Formula (4') rearranges to position 4. Therefore, optimal conditions among the deprotection reaction conditions that will be explained below may be adopted to reduce by-production of the isomer (4') and obtained the 3-isopropenyl-6-heptenal compound (4) with a good yield.

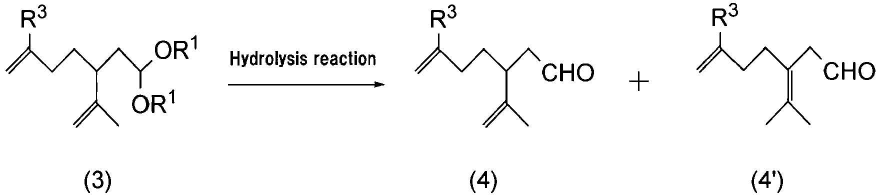

The 3-isopropenyl-6-heptenal compound (4) may be synthesized by adding acid and water, and if necessary, a solvent to the 3-isopropenyl-6-heptenal acetal compound (3), followed by cooling or heating to hydrolyze the acetal of the 3-isopropenyl-6-heptenal acetal compound (3).

Examples of the acid used in the hydrolysis include inorganic acids such as hydrochloric acid, hydrobromic acid, hydroiodic acid, sulfuric acid, nitric acid, phosphoric acid, or salts thereof; organic acids such as formic acid, acetic acid, propionic acid, oxalic acid, trifluoroacetic acid, methanesulfonic acid, benzenesulfonic acid, *p-*toluenesulfonic acid, naphthalenesulfonic acid, or salts thereof; Lewis acids such as lithium tetrafluoroborate, boron trifluoride, boron trichloride, boron tribromide, aluminum trichloride, zinc chloride, zinc bromide, zinc iodide, tin tetrachloride, tin tetrabromide, tin dichloride, titanium tetrachloride, titanium tetrabromide, and trimethylsilyl iodide; oxides such as alumina, silica gel, and titania; and minerals such as montmorillonite. The acid may be used alone or in combination thereof.

The amount of the acid used may be arbitrarily set as long as a practically sufficient reaction rate is achieved. The amount is preferably as low as possible in view of economy, and is preferably 0.00001 mol to 10,000 mol, more preferably 0.0001 mol to 1,000 mol, and even more preferably 0.001 mol to 100 mol, per mol of the substrate, 3-isopropenyl-6-heptenal acetal compound (3).

It is advantageous to add a larger amount of water in the hydrolysis because more water causes the equilibrium to tend toward aldehyde production, and the amount of water is preferably 1 mol to 10,000 mol, more preferably 1 mol to 1,000 mol, and even more preferably 1 mol to 500 mol, per mol of the substrate, 3-isopropenyl-6-heptenal acetal compound (3). By using said preferred amount, said more preferred amount, and said even more preferred amount, a preferred economy, workability, and/or yield, more preferred economy, workability, and/or yield, and even more preferred economy, workability, and/or yield may be ensured. The reaction may be carried out while removing alcohol produced by the hydrolysis of acetal from the system by a process such as distilling off and/or layer separation.

Examples of the solvent of the hydrolysis include ethers such as diethyl ether, dibutyl ether, tetrahydrofuran, and 1,4-dioxane; hydrocarbons such as hexane, heptane, benzene, toluene, xylene, and cumene; chlorinated solvents such as methylene chloride, chloroform, and trichloroethylene; ketones such as acetone and methyl ethyl ketone; aprotic polar solvents such as *N,N*-dimethylformamide (DMF), 1,3-dimethyl-2-imidazolidinone (DMI), dimethyl sulfoxide (DMSO), and hexamethylphosphoric triamide (HMPA); nitriles such as acetonitrile and propionitrile; esters such as ethyl acetate and n-butyl acetate; and alcohols such as methanol, ethanol, and t-butyl alcohol. The solvent may be used alone or in combination thereof.

The amount of the solvent used in the deprotection is preferably 10 g to 10,000 g, per mol of the substrate, 3-isopropenyl-6-heptenal acetal compound (3).

The reaction temperature of the hydrolysis varies, depending on the reaction conditions to be adopted, and is -78°C to 160°C, preferably -50°C to 140°C, more preferably -30°C to 120°C, and particularly preferably 0°C to 50°C.

The reaction time of the hydrolysis may be arbitrarily set. In view of the yield, it is desirable to monitor progress of the reaction with gas chromatography (GC) and/or silica gel thin layer chromatography (TLC) to complete the reaction. The reaction time is typically about 0.5 to 240 hours. When the target compound, 3-isopropenyl-6-heptenal compound (4) has a sufficient purity, the crude product may be used as such in a subsequent step. Alternatively, the crude product may be suitably purified by any purification method used in usual organic synthesis, such as distillation or various chromatography.

### [3] Step D

A step of preparing a 6-isopropenyl-3-methyl-3,9-decadien-1-ol compound (5) will be explained in detail below. The 6-isopropenyl-3-methyl-3,9-decadien-1-ol compound (5) may be formed as shown in the following reaction formula by subjecting the 3-isopropenyl-6-heptenal compound (4) obtained in Step C to a four-carbon homologation reaction including a Wittig reaction with an ethyltriphenylphosphonium halide compound (9) and ethylene oxide (10).

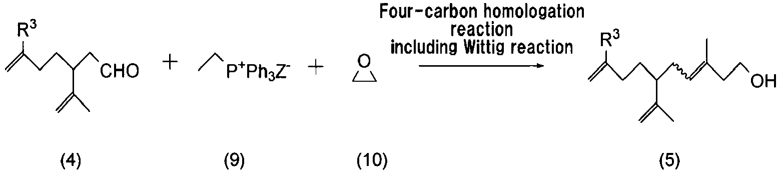

The ethyltriphenylphosphonium halide compound of the following general formula (9) will be explained. 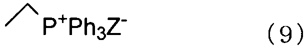 Z represents a halogen atom, preferably a chlorine atom, a bromine atom, or an iodine atom. The ethyltriphenylphosphonium halide compound (9) may be a commercially available one, or may be prepared by subjecting an ethyl halide compound of the following general formula (11) to a reaction with triphenylphosphine in a solvent.

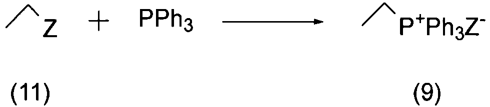

In the preparation of an ethyltriphenylphosphonium halide compound (9) by reacting an ethyl halide compound (11) with triphenylphosphine in a solvent, a metal halide and/or a quaternary onium salt may be added to accelerate the reaction. Examples of the metal halide include lithium iodide, sodium iodide, potassium iodide, lithium bromide, sodium bromide, and potassium bromide. Examples of the quaternary onium salt include tetraethylammonium bromide, tetrabutylammonium bromide, tetrabutylphosphonium bromide, tetraethylammonium iodide, tetrabutylammonium iodide, and tetrabutylphosphonium iodide.

In the preparation of the ethyltriphenylphosphonium halide compound (9), the reaction mixture may be made basic with a pH of, for example, 8 or more, by adding bicarbonates such as lithium bicarbonate, sodium bicarbonate, and potassium bicarbonate; carbonates such as lithium carbonate, sodium carbonate, and potassium carbonate; hydroxide salts such as lithium hydroxide, sodium hydroxide, and potassium hydroxide; and organic bases such as triethylamine, diisopropylethylamine, tributylamine, *N,N*-dimethylaniline, *N,N*-diethylaniline, pyridine, 4-dimethylaminopyridine, quinoline, pyrrolidine, piperidine, collidine, lutidine, and morpholine.

The solvent that is used in the preparation of the ethyltriphenylphosphonium halide compound (9) may be the same as the solvent of a Wittig reaction that will be explained below.

The amount of the solvent used in the preparation is preferably 10 g to 10,000 g, per mol of the halide.

The reaction temperature of the preparation of the ethyltriphenylphosphonium halide compound (9) varies, depending on the reaction conditions, and is -10°C to 180°C, preferably 0°C to 160°C, and more preferably 10°C to 140°C.

The reaction time of the preparation of the ethyltriphenylphosphonium halide compound (9) may be arbitrarily set. In view of the yield, it is desirable to monitor progress of the reaction with gas chromatography (GC) and/or silica gel thin layer chromatography (TLC) to complete the reaction. The reaction time is typically about 0.5 to 60 hours.

A 6-isopropenyl-3-methyl-3,9-decadien-1-ol compound of the following general formula (5) will be explained.

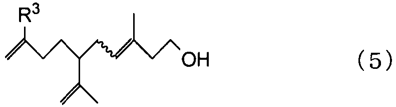

The 6-isopropenyl-3-methyl-3,9-decadien-1-ol compound (5) may include a (3*Z*,6*R*)-6-isopropenyl-3-methyl-3,9-decadien-1-ol compound of the following formula (5a), a (3*E*,6*R*)-6-isopropenyl-3-methyl-3,9-decadien-1-ol compound of the following formula (5b), a (3*Z*,6*S*)-6-isopropenyl-3-methyl-3,9-decadien-1-ol compound of the following formula (5c), and a (3*E*,6*S*)-6-isopropenyl-3-methyl-3,9-decadien-1-ol compound of the following formula (5d). These isomers may be used alone or in combination thereof, and preferably include, for example, the aforesaid compound (5a), which has the same skeleton as the natural sex pheromones of females of the California red scale and the White peach scale. 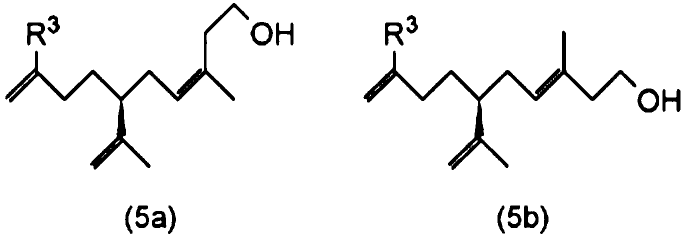 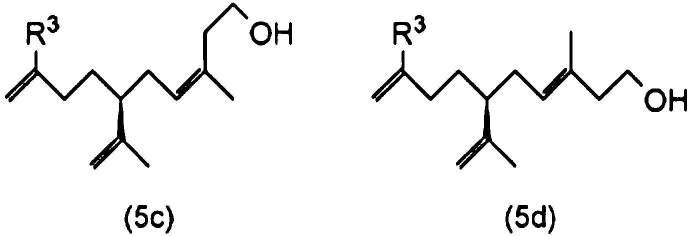 R³ is as defined for general formula (2) above.

A four-carbon homologation reaction including a Wittig reaction will be explained for an example of the number of carbon atoms when R³ is a hydrogen atom. The carbon atoms of the R³ moiety is one greater when R³ is a methyl group.

A four-carbon homologation reaction including a Wittig reaction may include the following two processes. Specifically:
as shown in the following Reaction Route 1, an ethyltriphenylphosphonium halide compound (9) having two carbon (C2) units is reacted with a base to convert it into an ylide (12). The ylide (12) is then reacted with ethylene oxide (10) having two carbons (C2) to prepare 3-triphenylphosphoniobutoxide (13) having a four-carbon skeleton (C4) (C2 + C2 = C4). Then, 3-triphenylphosphoniobutoxide (13) thus obtained is reacted with a base to convert it into phosphorus ylide (14), followed by subjecting the phosphorus ylide (14) to a Wittig reaction with a 3-isopropenyl-6-heptenal compound (4) having ten carbons (C10) to prepare a 6-isopropenyl-3-methyl-3,9-decadien-1-ol compound having fourteen carbons (C14) (5: R³ = hydrogen atom) (C4 + C10 = C14); and
as shown in the following Reaction Route 2, an ethyltriphenylphosphonium halide compound (9) having two carbon (C2) units is reacted with a base to prepare ylide (12). The ylide (12) is reacted with a 3-isopropenyl-6-heptenal compound (4) having ten carbons (C10) to prepare an intermediate, betaine (15), having a twelve-carbon skeleton (C12) (C2 + C10 = C12). The intermediate, betaine (15), thus obtained is then reacted with a base to prepare β-oxidophosphorus ylide (16), followed by reacting the β-oxidophosphorus ylide (16) with an ethylene oxide (10) having two carbons (C2) to prepare a 6-isopropenyl-3-methyl-3,9-decadien-1-ol compound having fourteen carbons (C14) (5: R³ = hydrogen atom) (C12 + C2 = C14). The process for synthesizing Reaction Route 2 is generally known as a SCOOPY reaction.

Among these Reaction Routes, an industrially practical process for synthesizing may be selected in view of the economy, reactivity, and/or yield. For example, Reaction Route 1 is preferred. By using said Reaction Route 1, a preferred yield may be ensured.

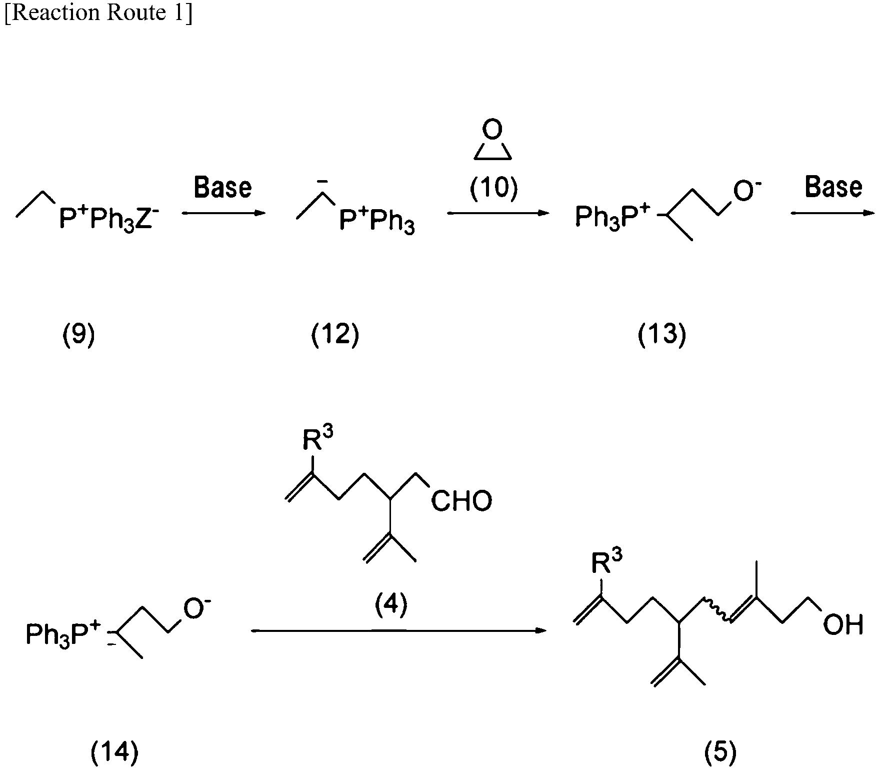

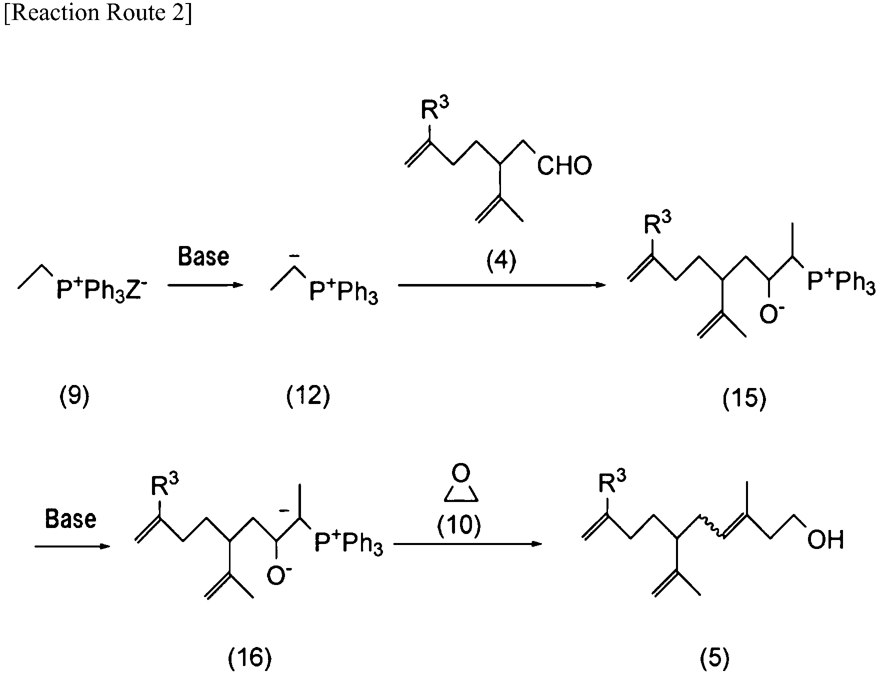

First, Reaction Route 1 will be explained in detail below.

In the ethyltriphenylphosphonium halide compound (9), Z represents a halogen atom.

The step of preparing ylide (12) from an ethyltriphenylphosphonium halide compound (9) having two carbon (C2) units may be carried out by adding a base to the ethyltriphenylphosphonium halide compound (9) in a solvent, if necessary, under heating or cooling so that the reaction temperature is within the reaction temperature described below.

Examples of the base used in the preparation of ylide (12) include metal alkoxides such as sodium methoxide, sodium ethoxide, sodium t-butoxide, sodium t-amyloxide, lithium methoxide, lithium ethoxide, lithium t-butoxide, lithium t-amyloxide, potassium methoxide, potassium ethoxide, potassium t-butoxide, and potassium t-amyloxide; organometallic reagents such as methyllithium, ethyllithium, n-butyllithium, methylmagnesium chloride, and dimsyl sodium; metal amides such as sodium amide, lithium amide, lithium diisopropylamide, lithium hexamethyldisilazide, sodium hexamethyldisilazide, potassium hexamethyldisilazide, and lithium dicyclohexylamide; and metal hydrides such as sodium hydride, potassium hydride, and calcium hydride. These bases may be used alone or in combination, and may be selected in view of the type of substrate, reactivity, and/or selectivity.

The amount of the base used in the preparation of ylide (12) is preferably 0.5 mol to 5 mol, per mol of the ethyltriphenylphosphonium halide compound (9).

The solvent that is used in the preparation of ylide (12) may be the same as a solvent of a Wittig reaction that will be explained below.

The amount of the solvent used in the preparation of ylide (12) is preferably 10 g to 10,000 g, per mol of the ethyltriphenylphosphonium halide compound (9).

The reaction temperature of the preparation of ylide (12) is preferably -78°C to 50°C, and more preferably -78°C to 40°C.

The reaction time of the preparation of ylide (12) is preferably 5 minutes to 18 hours, and more preferably 5 minutes to 10 hours. By using said more preferred reaction time, a more preferred reagent stability may be ensured.

The ylide (12) thus obtained may be subjected to two-carbon homologation with an ethylene oxide (10) having two carbons (C2) to prepare 3-triphenylphosphoniobutoxide (13) having a four-carbon skeleton (C4).

The amount of ethylene oxide (10) used in the two-carbon homologation reaction is 0.6 mol to 5 mol, per mol of the ethyltriphenylphosphonium halide compound (9).

The solvent of the two-carbon homologation reaction may be the same as a solvent of a Wittig reaction that will be explained below.

The amount of the solvent of the two-carbon homologation reaction is preferably 10 g to 10,000 g, per mol of the ethyltriphenylphosphonium halide compound (9).

The reaction temperature of the two-carbon homologation reaction is preferably -78°C to 50°C, and more preferably -78°C to 40°C.

The reaction time of the two-carbon homologation reaction is preferably 5 minutes to 18 hours, and more preferably 5 minutes to 10 hours. By using said more preferred reaction time, a more preferred reagent stability may be ensured.

3-Triphenylphosphoniobutoxide (13) obtained in the two-carbon homologation reaction is reacted with a base in a solvent under heating or cooling, if necessary, so that the reaction temperature is within the reaction temperature mentioned in the aforesaid step of preparing the ylide (12), to convert it into phosphorus ylide (14).

In the process for preparing phosphorus ylide (14) from 3-triphenylphosphoniobutoxide (13), the conditions of the base used, amount of base used, solvent, reaction temperature, and reaction time are the same as those mentioned in the aforesaid step of preparing the ylide (12).

Next, the phosphorus ylide (14) thus obtained may be subjected to a Wittig reaction with a 3-isopropenyl-6-heptenal compound (4) to prepare a 6-isopropenyl-3-methyl-3,9-decadien-1-ol compound (5).

The amount of the 3-isopropenyl-6-heptenal compound (4) used in the Wittig reaction is 0.1 mol to 5 mol, per mol of the ethyltriphenylphosphonium halide compound (9).

Examples of the solvent of the Wittig reaction include ethers such as diethyl ether, dibutyl ether, tetrahydrofuran, and 1,4-dioxane; hydrocarbons such as hexane, heptane, benzene, toluene, xylene, and cumene; chlorinated solvents such as methylene chloride, chloroform, and trichloroethylene; aprotic polar solvents such as *N,N-*dimethylformamide, 1,3-dimethyl-2-imidazolidinone, dimethyl sulfoxide, and hexamethylphosphoric triamide; nitriles such as acetonitrile and propionitrile; esters such as ethyl acetate and n-butyl acetate; and alcohols such as methanol, ethanol, and *t*-butyl alcohol. These solvents may be used alone or in combination.

The amount of the solvent used in the Wittig reaction is preferably 10 g to 10,000 g, per mol of the ethyltriphenylphosphonium halide compound (9).

The reaction temperature of the Wittig reaction is preferably -78°C to 50°C, and more preferably -50°C to 40°C.

The reaction time of the Wittig reaction may be arbitrarily set. In view of the yield, it is desirable to monitor progress of the reaction with gas chromatography (GC) and/or silica gel thin layer chromatography (TLC) to complete the reaction. The reaction time is typically about 0.5 to 24 hours.

Next, Reaction Route 2 will be explained in detail below.

In the process for reacting an ethyltriphenylphosphonium halide compound (9) with a base to prepare phosphorus ylide (12), the conditions of the base used, amount of base used, solvent, reaction temperature, and reaction time are the same as the conditions mentioned in the aforesaid step of preparing ylide (12).

Next, the ylide (12) thus obtained is reacted with a 3-isopropenyl-6-heptenal compound (4) to prepare an intermediate, betaine (15).

The amount of the 3-isopropenyl-6-heptenal compound (4) used in the preparation of the intermediate, betaine (15), is 0.1 mol to 5 mol, per mol of the ethyltriphenylphosphonium halide compound (9).

Examples of the solvent used in the preparation of the intermediate, betaine (15), include ethers such as diethyl ether, dibutyl ether, tetrahydrofuran, and 1,4-dioxane; hydrocarbons such as hexane, heptane, benzene, toluene, xylene, and cumene; chlorinated solvents such as methylene chloride, chloroform, and trichloroethylene; aprotic polar solvents such as *N,N*-dimethylformamide, 1,3-dimethyl-2-imidazolidinone, dimethyl sulfoxide, and hexamethylphosphoric triamide; nitriles such as acetonitrile and propionitrile; esters such as ethyl acetate and n-butyl acetate; and alcohols such as methanol, ethanol, and t-butyl alcohol. These solvents may be used alone or in combination.

The amount of the solvent used in the preparation of the intermediate, betaine (15), is preferably 10 g to 10,000 g, per mol of the ethyltriphenylphosphonium halide compound (9).

The reaction temperature of the preparation of the intermediate, betaine (15), is preferably -78°C to 50°C, and more preferably -78°C to 20°C.

The reaction time of the preparation of the intermediate, betaine (15), may be arbitrarily set, and is typically about 0.001 to 24 hours.

The intermediate, betaine (15), thus obtained is then reacted with a base to prepare β-oxidophosphorus ylide (16).

The conditions of the preparation of β-oxidophosphorus ylide (16) such as the base used, amount of base used, solvent, reaction temperature, and reaction time are the same as those mentioned in the aforesaid step of preparing ylide (12).

The β-oxidophosphorus ylide (16) thus obtained may be then subjected to a two-carbon homologation reaction with ethylene oxide (10) to prepare a 6-isopropenyl-3-methyl-3,9-decadien-1-ol compound (5).

The conditions of the two-carbon homologation reaction such as the amount of ethylene oxide (10) used, solvent, reaction temperature, and reaction time are the same as those mentioned in the aforesaid step of preparing 3-triphenylphosphoniobutoxide (13).

When the 6-isopropenyl-3-methyl-3,9-decadien-1-ol compound (5) thus obtained has a sufficient purity, the crude product may be used as such in a subsequent step. Alternatively, the crude product may be suitably purified by any purification method used in usual organic synthesis, such as distillation and/or various chromatography.

### [4] Step E

A step of preparing a 6-isopropenyl-3-methyl-3,9-decadienyl carboxylate compound (6) will be explained in detail below. A 6-isopropenyl-3-methyl-3,9-decadienyl carboxylate compound (6) may be formed by subjecting the 6-isopropenyl-3-methyl-3,9-decadien-1-ol compound (5) obtained in Step D to an esterification reaction as shown in the following reaction formula:

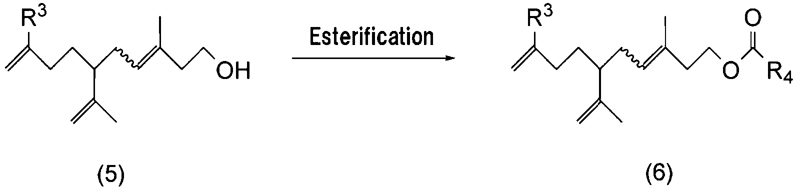

A 6-isopropenyl-3-methyl-3,9-decadienyl carboxylate compound of the following general formula (6) will be explained below.

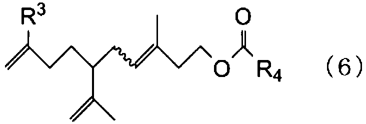

R³ is as defined for general formula (2) above, R⁴ represents a hydrogen atom or a monovalent hydrocarbon group having 1 to 10 carbon atoms, and the wavy line represents the E-form, the Z-form, or a mixture thereof.

Examples of the monovalent hydrocarbon group of R⁴ include linear saturated hydrocarbon groups such as a methyl group, an ethyl group, an n-propyl group, an n-butyl group, and an n-pentyl group; branched saturated hydrocarbon groups such as a 1-methylethyl group; linear unsaturated hydrocarbon groups such as a vinyl group, a 1-propenyl group, an ethynyl group, a propynyl group, and a 1-butynyl group; branched unsaturated hydrocarbon groups such as a 2-propenyl group and a 2-methyl-1-propenyl group; and isomers thereof. A part of the hydrogen atoms of the monovalent hydrocarbon group may be substituted with a methyl group or an ethyl group.

Specific examples of the 6-isopropenyl-3-methyl-3,9-decadienyl carboxylate compound (6) include 6-isopropenyl-3-methyl-3,9-decadienyl acetate (R³ = hydrogen atom; R⁴ = methyl group), 6-isopropenyl-3-methyl-3,9-decadienyl propionate (R³ = hydrogen atom; R⁴ = n-propyl group), 6-isopropenyl-3,9-dimethyl-3,9-decadienyl acetate (R³ = methyl group; R⁴ = methyl group), and 6-isopropenyl-3,9-dimethyl-3,9-decadienyl propionate (R³ = methyl group; R⁴ = n-propyl group).

The 6-isopropenyl-3-methyl-3,9-decadienyl carboxylate compound (6) may include a (3Z,6R)-6-isopropenyl-3-methyl-3,9-decadienyl carboxylate compound of the following formula (6a), a (3E,6R)-6-isopropenyl-3-methyl-3,9-decadienyl carboxylate compound of the following formula (6b), a (3Z,65)-6-isopropenyl-3-methyl-3,9-decadienyl carboxylate compound of the following formula (6c), and a (3E,6S)-6-isopropenyl-3-methyl-3,9-decadienyl carboxylate compound of the following formula (6d). These isomers may be used alone or in combination thereof, and preferably include, for example, the aforesaid compound (6a), which has the same skeleton as the natural sex pheromones of females of the California red scale and the White peach scale. 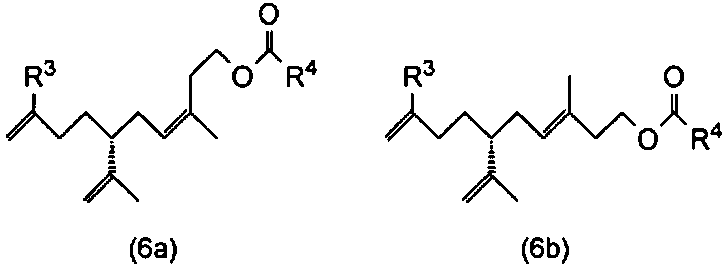 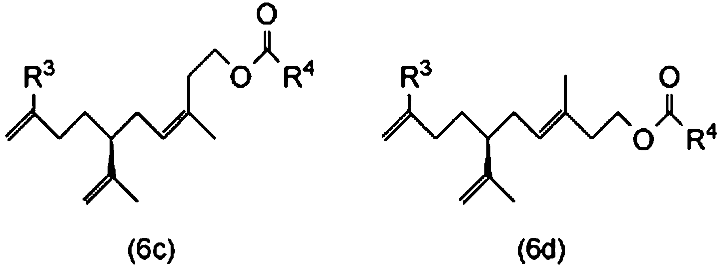 R³ and R⁴ are as defined for general formula (6) above.

The esterification reaction may be carried out by any known ester preparation process, such as (i) reacting with an acylating agent, (ii) reacting with a carboxylic acid, (iii) transesterification, and (iv) a process of converting a hydroxy group of a 6-isopropenyl-3-methyl-3,9-decadien-1-ol compound (5) into a leaving group and then reacting with a carboxylic acid.

### (i) Reaction with an acylating agent

The reaction with an acylating agent may be carried out by reacting a 6-isopropenyl-3-methyl-3,9-decadien-1-ol compound (5) with an acylating agent in a solvent or a mixture of two or more solvents in the presence of a base. The base may be added to the reaction system simultaneously with the acylating agent, or the acylating agent and then the base may be added to the reaction system in this order, or the base and then the acylating agent may be added to the reaction system in this order.

Examples of the acylating agent include acyl halides such as acyl chloride and acyl bromide; carboxylic anhydride such as acetic anhydride, and propionic anhydride; mixed carboxylic anhydrides such as mixed carboxylic/trifluoroacetic anhydride, mixed carboxylic/methanesulfonic anhydride, mixed carboxylic/trifluoromethanesulfonic anhydride, mixed carboxylic/benzensulfonic anhydride, and carboxylic/p-toluenesulfonic anhydride; and *p*-nitrophenyl carboxylate.

Specific examples of the acylating agent include acetyl chloride, propionyl chloride, acetic anhydride, and propionic anhydride.

The amount of the acylating agent used is preferably 1 mol to 500 mol, more preferably 1 mol to 50 mol, and even more preferably 1 mol to 5 mol, per mol of the 6-isopropenyl-3-methyl-3,9-decadien-1-ol compound (5).

Examples of the base used in the reaction with the acylating agent include *N,N-*diisopropylethylamine, N,N-dimethylaniline, N,N-diethylaniline, pyridine, 2-ethyl pyridine, and 4-dimethylaminopyridine.

The amount of the base used is 1 mol to 500 mol, per mol of the 6-isopropenyl-3-methyl-3,9-decadien-1-ol compound (5).

Examples of the solvent used in the reaction with the acylating agent include the aforesaid base used as a solvent, or chlorinated solvents such as methylene chloride, chloroform, and trichloroethylene; hydrocarbons such as hexane, heptane, benzene, toluene, xylene, and cumene; ethers such as diethyl ether, dibutyl ether, diethyleneglycol diethyl ether, diethyleneglycol dimethyl ether, tetrahydrofuran, and 1,4-dioxane; nitriles such as acetonitrile; ketones such as acetone and 2-butanone; esters such as ethyl acetate and n-butyl acetate; and aprotic polar solvents such as *N,N-*dimethylformamide, dimethyl sulfoxide, and hexamethylphosphoric triamide. These solvents may be used alone or in combination thereof.

The amount of the solvent used in the reaction with the acylating agent is preferably 10 g to 1,000,000 g, per mol of the 6-isopropenyl-3-methyl-3,9-decadien-1-ol compound (5).

A reaction with an acylating agent such as carboxylic anhydride, mixed carboxylic anhydride, and p-nitrophenyl carboxylate may be carried out in the presence of an acid catalyst instead of a base.

Examples of the acid catalyst include inorganic acids such as hydrochloric acid, hydrobromic acid, sulfuric acid, and nitric acid; organic acids such as oxalic acid, trifluoroacetic acid, methanesulfonic acid, benzenesulfonic acid, and p-toluenesulfonic acid; Lewis acids such as aluminum trichloride, aluminum ethoxide, aluminum isopropoxide, aluminum oxide, boron trifluoride, boron trichloride, boron tribromide, magnesium chloride, magnesium bromide, magnesium iodide, zinc chloride, zinc bromide, zinc iodide, tin tetrachloride, tin tetrabromide, dibutyltin dichloride, dibutyltin dimethoxide, dibutyltin oxide, titanium tetrachloride, titanium tetrabromide, titanium(IV) methoxide, titanium(IV) ethoxide, titanium(IV) isopropoxide, and titanium(IV) oxide; and cation exchange resins. These acid catalysts may be used alone or in combination thereof.

The amount of the acid catalyst used in a reaction with acylating agents such as carboxylic anhydride, mixed carboxylic anhydride, and p-nitrophenyl carboxylate is preferably 0.0001 mol to 100 mol.

An appropriate reaction temperature of the reaction with the acylating agent may be selected according to the type of acylating agent used and/or reaction conditions, and typically is preferably from -50°C up to the boiling point of the solvent, and more preferably from -20°C up to a room temperature. A room temperature is, for example, 5°C to 35°C (the same applies hereinafter).

The reaction time of the reaction with the acylating agent may be arbitrarily set. The reaction time is preferably optimized by monitoring progress of the reaction with gas chromatography (GC) and/or thin layer chromatography (TLC). The reaction time is typically and preferably 5 minutes to 240 hours.

### (ii) Reaction with carboxylic acid

The reaction with a carboxylic acid is a dehydration reaction of the 6-isopropenyl-3-methyl-3,9-decadien-1-ol compound (5) with a carboxylic acid, and is typically carried out in the presence of an acid catalyst.

Specific examples of the carboxylic acid in the reaction of the 6-isopropenyl-3-methyl-3,9-decadien-1-ol compound (5) with a carboxylic acid include acetic acid and propionic acid.

The amount of carboxylic acid used is preferably 1 mol to 500 mol, more preferably 1 mol to 50 mol, and even more preferably 1 mol to 5 mol, per mol of the 6-isopropenyl-3-methyl-3,9-decadien-1-ol compound (5).

When a reaction of the 6-isopropenyl-3-methyl-3,9-decadien-1-ol compound (5) with a carboxylic acid is carried out, the reaction may be carried out in the presence of an acid catalyst. The acid catalyst is the same as the aforesaid acid catalyst used in the reaction with the acylating agent.

The amount of the acid catalyst used is preferably 0.0001 mol to 100 mol, more preferably 0.001 mol to 1 mol, and even more preferably 0.01 mol to 0.05 mol, per mol of the 6-isopropenyl-3-methyl-3,9-decadien-1-ol compound (5).

The solvent used and the amount of the solvent used in the reaction of the 6-isopropenyl-3-methyl-3,9-decadien-1-ol compound (5) with a carboxylic acid are the same as those of the aforesaid reaction with the acylating agent.

An appropriate reaction temperature of the reaction of the 6-isopropenyl-3-methyl-3,9-decadien-1-ol compound (5) with a carboxylic acid may be selected according to the reaction conditions, and typically is preferably from -50°C up to the boiling point of the solvent, and more preferably from a room temperature up to the boiling point of the solvent.

The reaction may be carried out in a solvent including hydrocarbons such as hexane, heptane, benzene, toluene, xylene, and cumene, while removing the resulting water from the system by azeotropic distillation. In such a case, water may be distilled off with reflux at the boiling point of the solvent at atmospheric pressure, or water may be distilled off at a reduced pressure at a lower temperature than the boiling point.

The reaction time of the reaction with a carboxylic acid may be arbitrarily set. The reaction time may be optimized by monitoring progress of the reaction with gas chromatography (GC) and/or thin layer chromatography (TLC). The reaction time is typically and preferably 5 minutes to 240 hours.

### (iii) Transesterification

Transesterification is carried out by reacting a 6-isopropenyl-3-methyl-3,9-decadien-1-ol compound (5) with an alkyl carboxylate in the presence of a catalyst and by removing the resulting alcohol.

The alkyl carboxylate is preferably a primary alkyl ester of a carboxylic acid. More preferred are methyl carboxylate, ethyl carboxylate, and n-propyl carboxylate. By using said methyl carboxylate, ethyl carboxylate, and n-propyl carboxylate, a more preferred price and easier progress of the reaction may be ensured.

Specific examples of the alkyl carboxylate include, but are not limited to, methyl acetate, ethyl acetate, propyl acetate, methyl propionate, ethyl propionate, and propyl propionate.

The amount of the alkyl carboxylate used is preferably 1 mol to 500 mol, more preferably 1 mol to 50 mol, and even more preferably 1 mol to 5 mol, per mol of the 6-isopropenyl-3-methyl-3,9-decadien-1-ol compound (5).

Examples of the catalyst used in the transesterification include inorganic acids such as a hydrochloric acid, hydrobromic acid, sulfuric acid, and nitric acid; organic acids such as oxalic acid, trifluoroacetic acid, methanesulfonic acid, benzenesulfonic acid, and p-toluenesulfonic acid; bases such as sodium methoxide, sodium ethoxide, potassium t-butoxide, and 4-dimethylaminopyridine; salts such as sodium cyanide, potassium cyanide, sodium acetate, potassium acetate, calcium acetate, tin acetate, aluminum acetate, aluminum acetoacetate, and alumina; and Lewis acids such as aluminum trichloride, aluminum ethoxide, aluminum isopropoxide, aluminum oxide, boron trifluoride, boron trichloride, boron tribromide, magnesium chloride, magnesium bromide, magnesium iodide, zinc chloride, zinc bromide, zinc iodide, tin tetrachloride, tin tetrabromide, dibutyltin dichloride, dibutyltin dimethoxide, dibutyltin oxide, titanium tetrachloride, titanium tetrabromide, titanium(IV) methoxide, titanium(IV) ethoxide, titanium(IV) isopropoxide, and titanium(IV) oxide. These catalysts may be used alone or in combination thereof.

The amount of the catalyst used is preferably 0.0001 mol to 100 mol, more preferably 0.001 mol to 1 mol, and even more preferably 0.01 mol to 0.05 mol, per mol of the 6-isopropenyl-3-methyl-3,9-decadien-1-ol compound (5).

The transesterification may be carried out without a solvent by using the alkyl carboxylate itself, which is a reagent, as the solvent, so that the transesterification may be carried out without extra operations such as concentration and/or solvent recovery. The transesterification is therefore preferably carried out without a solvent, or may be carried out with a supplemental solvent.

Examples of the solvent used in the transesterification include hydrocarbons such as hexane, heptane, benzene, toluene, xylene, and cumene; and ethers such as diethyl ether, dibutyl ether, diethylene glycol diethyl ether, diethylene glycol dimethyl ether, tetrahydrofuran, and 1,4-dioxane. The solvent may be used alone or in combination thereof.

The amount of the solvent used in the transesterification is preferably 10 g to 1,000,000 g, per mol of the 6-isopropenyl-3-methyl-3,9-decadien-1-ol compound (5).

An appropriate reaction temperature of the transesterification may be selected according to the type of alkyl carboxylate used and/or reaction conditions. The transesterification typically is carried out under heat. In view of easier progress of the reaction, the transesterification is preferably carried out around a boiling point of a lower alcohol, such as methanol or ethanol, that is generated in the reaction, has 1 to 3 carbon atoms, and has a low boiling point, while distilling the lower alcohol off. The alcohol may be distilled off at a reduced pressure at a lower temperature than the boiling point.

The reaction time of the transesterification may be arbitrarily set. The reaction time may be optimized by monitoring progress of the reaction with gas chromatography (GC) and/or thin layer chromatography (TLC), and is typically and preferably 5 minutes to 240 hours.

### (iv) Reaction with carboxylic acid after conversion to leaving group

The process of converting a hydroxy group of a 6-isopropenyl-3-methyl-3,9-decadien-1-ol compound (5) into a leaving group and then reacting it with a carboxylic acid may be carried out by, for example, converting the 6-isopropenyl-3-methyl-3,9-decadien-1-ol compound (5) into a corresponding leaving group such as a halogen atom such as chloride, bromide, or iodide; an alkanesulfonyloxy group such as methanesulfonate or trifluoromethanesulfonate; or an arenesulfonyloxy such as benzenesulfonate or p-toluenesulfonate, and then reacting it with a carboxylic acid in a solvent in the presence of a base.

Examples of the carboxylic acid include the same compound as the carboxylic acid of the aforesaid reaction with a carboxylic acid.

The amount of the carboxylic acid used is preferably 1 mol to 500 mol, more preferably 1 mol to 50 mol, and even more preferably 1 mol to 5 mol, per mol of the 6-isopropenyl-3-methyl-3,9-decadien-1-ol compound (5).

The solvent that is used in the process of converting the 6-isopropenyl-3-methyl-3,9-decadien-1-ol compound (5) into a leaving group and then reacting it with a carboxylic acid, the amount of the solvent used, the base, the amount of the base used, the reaction time, and the reaction temperature may be the same as those mentioned in the reaction of the 6-isopropenyl-3-methyl-3,9-decadien-1-ol compound (5) with an acylating agent.

Instead of reacting the carboxylic acid in a solvent in the presence of a base, carboxylate salts such as sodium carboxylate, lithium carboxylate, potassium carboxylate, and ammonium carboxylate may be used. The amount of the carboxylate salt used may be the same as the amount of the carboxylic acid used in the esterification reaction with a carboxylic acid.

When the 6-isopropenyl-3-methyl-3,9-decadienyl carboxylate compound (6) obtained in the esterification reaction has a sufficient purity, the crude product may be used as such. Alternatively, the crude product may be suitably purified by any purification method used in usual organic synthesis, such as distillation and/or various chromatography, among which distillation is particularly preferred. By using said distillation, a particularly preferred industrial economy may be ensured.

### [5] Step A

Step A of synthesizing a 4-methyl-3-pentenal acetal compound (1) having a leaving group X at 5-position will be explained in detail below. A 4-methyl-3-pentenal acetal compound (1) having a leaving group X at 5-position may be formed by reacting a propyltriphenylphosphonium halide compound (7) having an acetal at 3-position with a base to convert it into phosphorus ylide (17), and then subjecting it to a Wittig reaction with a 2-propanone compound (8) having a leaving group X, as shown in the following reaction formula:

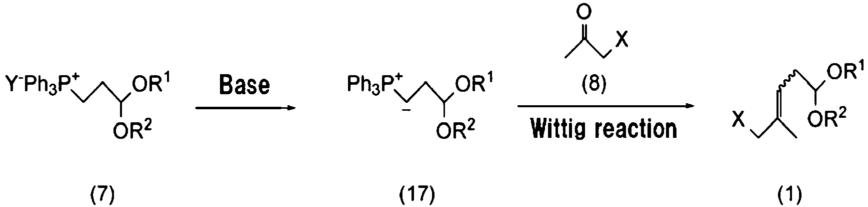

A propyltriphenylphosphonium halide compound having an acetal at 3-position of the following general formula (7) will be explained. 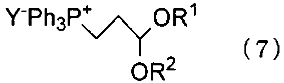
R¹ and R² are as defined for general formula (1) above.
Y represents a halogen atom and preferably represents a chlorine atom, a bromine atom, or an iodine atom.

Specific examples of the propyltriphenylphosphonium halide compound (7) having an acetal at 3-position include 3,3-dimethoxypropyltriphenylphosphonium chloride, 3,3-dimethoxypropyltriphenylphosphonium bromide, 3,3-diethoxypropyltriphenylphosphonium bromide, 2-(1,3-dioxolan-2-yl)ethyltriphenylphosphonium bromide, and 3,3-diethoxypropyltriphenylphosphonium iodide.

The propyltriphenylphosphonium halide compound (7) having an acetal at 3-position may be a commercially available one, or may be prepared by reacting a propyl halide compound having an acetal at 3-position of the following general formula (18) with triphenylphosphine in a solvent.

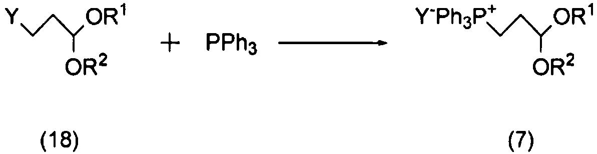

In the preparation of a propyltriphenylphosphonium halide compound (7) having an acetal at 3-position by reacting a propyl halide compound (18) having an acetal at 3-position with triphenylphosphine in a solvent, a metal halide and/or a quaternary onium salt may be added to accelerate the reaction. Examples of the metal halide include lithium iodide, sodium iodide, potassium iodide, lithium bromide, sodium bromide, and potassium bromide. Examples of the quaternary onium salt include tetraethylammonium bromide, tetrabutylammonium bromide, tetrabutylphosphonium bromide, tetraethylammonium iodide, tetrabutylammonium iodide, and tetrabutylphosphonium iodide.

In the preparation of a propyltriphenylphosphonium halide compound (7) having an acetal at 3-position, deprotection of an acetal may occur if the reaction mixture becomes acidic. To suppress such deprotection, the reaction mixture may be made basic by adding bicarbonates such as lithium bicarbonate, sodium bicarbonate, and potassium bicarbonate; carbonates such as lithium carbonate, sodium carbonate, and potassium carbonate; hydroxide salts such as lithium hydroxide, sodium hydroxide, and potassium hydroxide; and organic bases such as triethylamine, diisopropylethylamine, tributylamine, *N,N*-dimethylaniline, *N,N*-diethylaniline, pyridine, 4-dimethylaminopyridine, quinoline, pyrrolidine, piperidine, collidine, lutidine, and morpholine. Preferably, the reaction mixture has a pH of, for example, 8 or more. Potassium carbonate is particularly preferred. By using said potassium carbonate, a particularly preferred economy and/or isomerization suppression may be ensured.

The solvent used in the preparation of the propyltriphenylphosphonium halide compound (7) having an acetal at 3-position may be the same as solvents of preparation of phosphorus ylide (17) and a Wittig reaction that will be explained below.

The amount of the solvent used in the preparation is preferably 10 g to 10,000 g, per mol of the propyl halide compound (18) having an acetal at 3-position.

The reaction temperature of the preparation of the propyltriphenylphosphonium halide compound (7) having an acetal at 3-position varies, depending on reaction conditions, and is -10°C to 180°C, preferably 0°C to 160°C, and more preferably 10°C to 140°C.

The reaction time of the preparation of the propyltriphenylphosphonium halide compound (7) having an acetal at 3-position may be arbitrarily set. In view of the yield, it is desirable to monitor progress of the reaction with gas chromatography (GC) and/or silica gel thin layer chromatography (TLC) to complete the reaction. The reaction time is typically about 0.5 to 60 hours.

A 2-propanone compound having a leaving group X of the following general formula (8) will be explained. 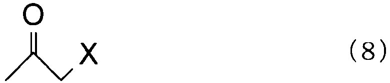 X is as defined for general formula (1) above.

The leaving group X is preferably an acetoxy group, a propionyloxy group, and a pivaloyloxy group. By using said acetoxy group, propionyloxy group, and pivaloyloxy group, a preferred reactivity and/or economy may be ensured.

The 2-propanone compound (8) having a leaving group X may be a commercially available one, or may be prepared from hydroxyacetone by any known process.

The Wittig reaction is carried out by reacting a propyltriphenylphosphonium halide compound (7) having an acetal at 3-position with a base in a solvent to form phosphorus ylide (17), which is then reacted with a 2-propanone compound (8) having a leaving group X under cooled or heated conditions, if necessary.

Examples of the base used in the phosphorus ylide preparation include metal alkoxides such as sodium methoxide, sodium ethoxide, sodium t-butoxide, sodium t-amyloxide, lithium methoxide, lithium ethoxide, lithium t-butoxide, lithium t-amyloxide, potassium methoxide, potassium ethoxide, potassium t-butoxide, and potassium t-amyloxide; organometallic reagents such as methyllithium, ethyllithium, n-butyllithium, methylmagnesium chloride, and dimsyl sodium; metal amides such as sodium amide, lithium amide, lithium diisopropylamide, lithium hexamethyldisilazide, sodium hexamethyldisilazide, potassium hexamethyldisilazide, and lithium dicyclohexylamide; and metal hydrides such as sodium hydride, potassium hydride, and calcium hydride. These bases may be used alone or in combination thereof, and may be selected in view of the type of substrate, reactivity, and/or selectivity.

The amount of the base used in the phosphorus ylide preparation is preferably 0.7 mol to 5 mol, per mol of the propyltriphenylphosphonium halide compound (7) having an acetal at 3-position.

The solvent used in the phosphorus ylide preparation may be the same as a solvent of a Wittig reaction that will be explained below.

The amount of the solvent used in the preparation of ylide (12) is preferably 10 g to 10,000 g, per mol of the propyltriphenylphosphonium halide compound (7) having an acetal at 3-position.

The reaction temperature of the phosphorus ylide preparation is preferably -78°C to 50°C, and more preferably -78°C to 40°C.

The reaction time of the phosphorus ylide preparation is preferably 5 minutes to 18 hours, and more preferably 5 minutes to 10 hours. By using said more preferred reaction time, a more preferred reagent stability may be ensured.

The amount of the 2-propanone compound (8) having a leaving group X used in the Wittig reaction is 0.6 mol to 5 mol, per mol of the propyltriphenylphosphonium halide compound (7) having an acetal at 3-position.

Examples of the solvent of the Wittig reaction include ethers such as diethyl ether, dibutyl ether, tetrahydrofuran, and 1,4-dioxane; hydrocarbons such as hexane, heptane, benzene, toluene, xylene, and cumene; chlorinated solvents such as methylene chloride, chloroform, and trichloroethylene; aprotic polar solvents such as *N,N-*dimethylformamide, 1,3-dimethyl-2-imidazolidinone, dimethyl sulfoxide, and hexamethylphosphoric triamide; nitriles such as acetonitrile and propionitrile; esters such as ethyl acetate and n-butyl acetate; and alcohols such as methanol, ethanol, and t-butyl alcohol. These solvents may be used alone or in combination.

The amount of the solvent used in the Wittig reaction is preferably 10 g to 10,000 g, per mol of the propyltriphenylphosphonium halide compound (7) having an acetal at 3-position.

The reaction temperature of the Wittig reaction is preferably -78°C to 50°C, and more preferably -50°C to 40°C.

The reaction time of the Wittig reaction may be arbitrarily set. In view of the yield, it is desirable to monitor progress of the reaction with gas chromatography (GC) and/or silica gel thin layer chromatography (TLC) to complete the reaction. The reaction time is typically about 0.5 to 24 hours.

When the 4-methyl-3-pentenal acetal compound (1) having a leaving group X at 5-position obtained in the Wittig reaction has a sufficient purity, the crude product may be used as such in a subsequent step. Alternatively, the crude product may be suitably purified in any purification method used in usual organic synthesis such as distillation and/or various chromatography. Distillation is particularly preferred. By using said distillation, a particularly preferred industrial economy may be ensured.

Thus, there is provided a process for efficiently preparing a 6-isopropenyl-3-methyl-3,9-decadienyl carboxylate compound (6), without an oxidation reaction and a reduction reaction that are industrially unfeasible in view of safety, economy, and/or environmental impact. There is also provided a process for preparing a 3-isopropenyl-6-heptenal compound (4), which is a useful intermediate for preparing 6-isopropenyl-3-methyl-3,9-decadienyl carboxylate compounds (6). There is also provided a process for preparing a 4-methyl-3-pentenal acetal compound (1) having a leaving group X at 5-position, which is a useful common intermediate for preparing 3-isopropenyl-6-heptenal compounds (4).

The aforesaid preparation processes are useful on an industrial basis because an oxidation reaction and a reduction reaction are unnecessary.

### EXAMPLES

The present invention will be described with reference to the following Examples. It should be noted that the present invention is not limited to or by the Examples.

The term "product ratio" means a ratio of area percentages in GC. The term "yield" is calculated from the area percentages determined by GC.

In the Examples, monitoring of the reactions and calculation of the yields were carried out in the following GC conditions.

GC conditions: GC: Capillary gas chromatograph GC-2014 (Shimadzu Corporation); column: DB-5, 0.25 µm x 0.25 mmϕ x 30 m; carrier gas: He (1.55 mL/min), detector: FID; column temperature: 100°C, elevated in a rate of 10°C/min, and up to 230°C.

The yield was calculated according to the following equation in consideration of purities (% GC) of a starting material and a product.

Yield (%) = {[(weight of a product obtained by a reaction × % GC) / molecular weight of a product] ÷ [(weight of a starting material in a reaction × % GC) / molecular weight of a starting material] × 100

The term "crude yield" refers to a yield of a crude product obtained without purification.

### Synthetic Example 1: 2-Propanone compound having leaving group X (8: X = acetoxy group)

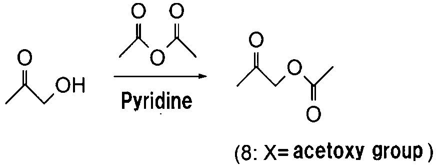

Hydroxyacetone (200 g: 2.595 mol) and pyridine (21.36 g: 0.270 mol) were placed in a reactor in a nitrogen atmosphere and then stirred at a room temperature. Acetic anhydride (330.77 g: 3.240 mol) was added dropwise to the reaction solution for 7 hours at 15 to 50°C. After the completion of the dropwise addition, the reaction mixture was stirred for 14 hours at a room temperature. The reaction mixture was then distilled at a reduced pressure to obtain a 2-propanone compound having an acetoxy group as a leaving group X (8: X = acetoxy group) (242.34 g: 2.079 mol). The yield calculated from all fractions including a fore-running fraction was 90.37%.

2-Propanone compound having leaving group X (8: X = acetoxy group):
IR (D-ATR): v = 2936, 1749, 1733, 1420, 1376, 1272, 1238, 1179, 1071, 969, 891, 824, 678, 608, 504, 457 cm⁻¹.
¹H-NMR (500 MHz, CDCl₃): 2.26 (3H, s), 2.27 (3H, s), 4.62 (2H, s) ppm.
¹³C-NMR (125 MHz, CDCl₃): δ = 20.37, 25.95, 68.21, 170.12, 201.47 ppm.
GC-MS (EI, 70 eV): 29, 43, 57, 65, 73, 86, 101, 116.

### Synthetic Example 2: 2-Propanone compound having leaving group X (8: X = pivaloyloxy group)

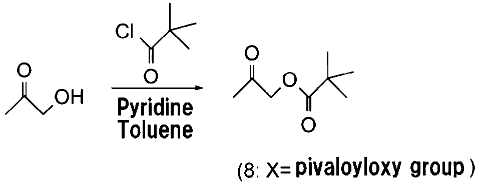

Hydroxyacetone (102.39 g: 1.382 mol), pyridine (327.95 g: 4.146 mol), and toluene (500 g) were placed in a reactor in a nitrogen atmosphere and then stirred at a room temperature. Pivaloyl chloride (200 g: 1.659 mol) was added dropwise to the reaction solution for 2 hours at 20 to 40°C. After the completion of the dropwise addition, the reaction mixture was stirred for 210 minutes at a room temperature. Purified water (700 g) was then added to the reaction solution, and the reaction solution was stirred for 30 minutes. After the completion of the stirring, the organic layer was separated. The organic layer thus separated was subjected to typical post-treatment of washing and then concentration to obtain a crude product (210.31 g) of 1-pivaloyloxy-2-propanone (8). The crude product of 1-pivaloyloxy-2-propanone (8) thus obtained was distilled at a reduced pressure to obtain a 2-propanone compound having a pivaloyloxy group as a leaving group X (8: X = pivaloyloxy group) (148.65 g: 0.933 mol). The yield calculated from all fractions including a fore-running fraction was 59.11%.

2-Propanone compound having leaving group X (8: X = pivaloyloxy group):
IR (D-ATR): v = 2976, 2936, 2875, 1730, 1481, 1462, 1418, 1463, 1419, 1397, 1367, 1287, 1229, 1191, 1144, 1052, 966, 895, 766, 636, 503, 419 cm⁻¹.
¹H-NMR (500 MHz, CDCl₃): 1.25 (9H, s), 2.13 (3H, s), 4.60 (2H, s) ppm.
¹³C-NMR (125 MHz, CDCl₃): δ = 26.04, 27.08, 38.65, 68.21, 177.77, 201.96 ppm.
GC-MS (EI, 70 eV): 29, 41, 57, 69, 85, 101, 115, 128, 143, 158.

### Example 1: Preparation of propyltriphenylphosphonium halide compound having acetal at 3-position (7: Y = bromine atom; R¹ and R² = ethylene group) and synthesis of 4-methyl-3-pentenal acetal compound having leaving group X at 5-position (1: X = acetoxy group; R¹ and R² = ethylene group)

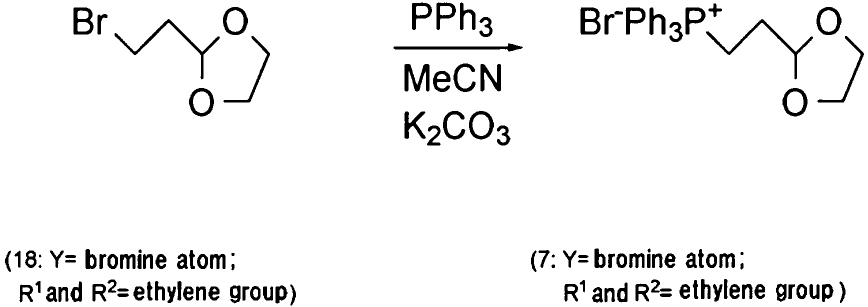

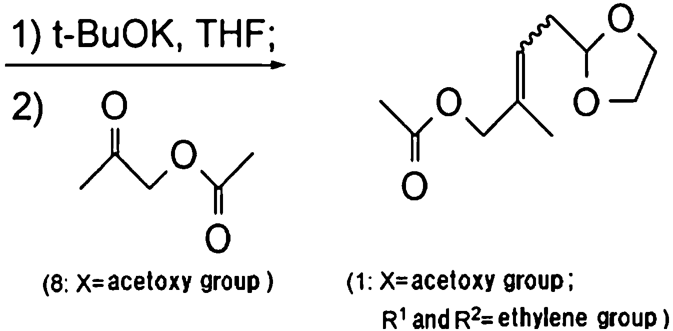

A propyl halide compound having an acetal at 3-position (18: Y = bromine atom; R¹ and R² = ethylene group) (30 g: 0.145 mol), triphenylphosphine (PPh₃) (45.72 g: 0.174 mol), potassium carbonate (K₂CO₃) (0.69 g: 0.005 mol), and acetonitrile (MeCN) (33.2 g) were placed in a reactor in a nitrogen atmosphere and then refluxed with stirring for 16 hours to prepare a propyltriphenylphosphonium halide compound having an acetal at 3-position (7: Y = bromine atom; R¹ and R² = ethylene group). The internal temperature of the reaction solution was cooled to a room temperature (20 to 25°C), and then tetrahydrofuran (THF) (132.8 g) was added to the reaction solution. The internal temperature was cooled to 0 to 5°C, then t-butoxy potassium (t-BuOK) (19.20 g: 0.171 mol) was added to the reaction mixture, and the reaction mixture was stirred for 4 hours. The internal temperature was then cooled to 4°C, and then a 2-propanone compound having a leaving group X (8: X = acetoxy group) (15.15 g: 0.131 mol) prepared as in Synthetic Example 1 was added dropwise for 40 minutes, followed by stirring the reaction mixture for 20 hours. Purified water (83 g) was then added to the reaction solution, and the reaction solution was stirred for 30 minutes. After the completion of the stirring, the organic layer was separated. The organic layer thus separated was distilled at a reduced pressure to obtain the target compound, 4-methyl-3-pentenal acetal compound having a leaving group X at 5-position (1: X = acetoxy group; R¹ and R² = ethylene group) (19.89 g: 0.091 mol). The yield calculated from all fractions including a fore-running fraction was 69.69%.

4-Methyl-3-pentenal acetal compound having leaving group X at 5-position (1: X = acetoxy group; R¹ and R² = ethylene group):
IR (D-ATR): v = 3515, 2974, 2887, 1738, 1436, 1369, 1233, 1137, 1024, 955, 863, 712, 607, 559 cm⁻¹.
¹H-NMR (500 MHz, CDCl₃): 1.77 (3H, s-like), 2.04 (3H, s), 2.43-2.37 (2H, m), 3.81-3.99 (4H, m), 4.58 (2H, s), 4.86 (1H, t, J = 4.8 Hz), 5.43-5.47 (1H, m) ppm.
¹³C-NMR (125 MHz, CDCl₃): δ = 14.17, 20.84, 21.52, 32.65, 63.10, 64.92, 103.67, 123.42, 133.27, 170.95 ppm.
GC-MS (EI, 70 eV): 29, 45, 55, 73, 83, 95, 113, 125, 139, 155, 176, 189, 199.

### Example 2: Synthesis of 4-methyl-3-pentenal acetal compound having leaving group X at 5-position (1: X = pivaloyloxy group; R¹ and R² = ethylene group)

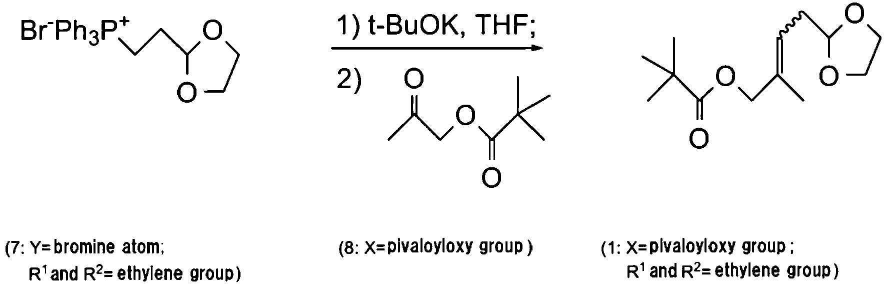

A propyltriphenylphosphonium halide compound having an acetal at 3-position (7: Y = bromine atom; R¹ and R² = ethylene group) (100 g: 0.225 mol) and tetrahydrofuran (THF) (225 g) were placed in a reactor in a nitrogen atmosphere, and then the internal temperature was cooled to 0 to 5°C. t-Butoxy potassium (t-BuOK) (25.27 g: 0.225 mol) was then added, and the reaction mixture was stirred for 1 hour. A 2-propanone compound having a leaving group X (8: X = pivaloyloxy group) (35.60 g: 0.202 mol) prepared as in Synthetic Example 2 was then added dropwise for 65 minutes, and then the reaction mixture was stirred for 19 hours. A solution of acetic acid (prepared from purified water (112.5 g) and acetic acid (2.7 g)) was then added to the reaction solution, and the reaction solution was stirred for 30 minutes. After the completion of the stirring, the organic layer was separated. The organic layer thus separated was subjected to typical post-treatment of washing and then concentration, and then the reaction mixture was distilled at a reduced pressure to obtain the target compound, 4-methyl-3-pentenal acetal compound having a pivaloyloxy group as a leaving group X at 5-position (1: X = pivaloyloxy group; R¹ and R² = ethylene group) (28.44 g: 0.114 mol). The yield calculated from all fractions including a fore-running fraction was 58.07%.

4-Methyl-3-pentenal acetal compound having leaving group X at 5-position (1: X = pivaloyloxy group; R¹ and R² = ethylene group):
IR (D-ATR): v = 2974, 2879, 1728, 1480, 1460, 1398, 1365, 1283, 1147, 1032, 962, 863, 770, 560 cm⁻¹.
¹H-NMR (500 MHz, CDCl₃): 1.19 (9H, s), 1.76 (3H, s-like), 2.42-2.48 (2H, m), 3.82-3.99 (4H, m), 4.58 (2H, s), 4.87 (1H, t, J = 4.8 Hz), 5.41-5.45 (1H, m) ppm.
¹³C-NMR (125 MHz, CDCl₃): δ = 14.03, 21.35, 27.15, 32.65, 38.79, 63.10, 64.92, 103.76, 122.82, 133.69, 178.36 ppm.
GC-MS (EI, 70 eV): 29, 45, 57, 73, 95, 113, 125, 139, 157, 169, 183, 195, 209, 221, 242.

### Example 3: Preparation of propyltriphenylphosphonium halide compound having acetal at 3-position (7: Y = bromine atom; R¹ = methyl group; R² = methyl group) and synthesis of 4-methyl-3-pentenal acetal compound having leaving group X at 5-position (1: X = acetoxy group; R¹ = methyl group; R² = methyl group)

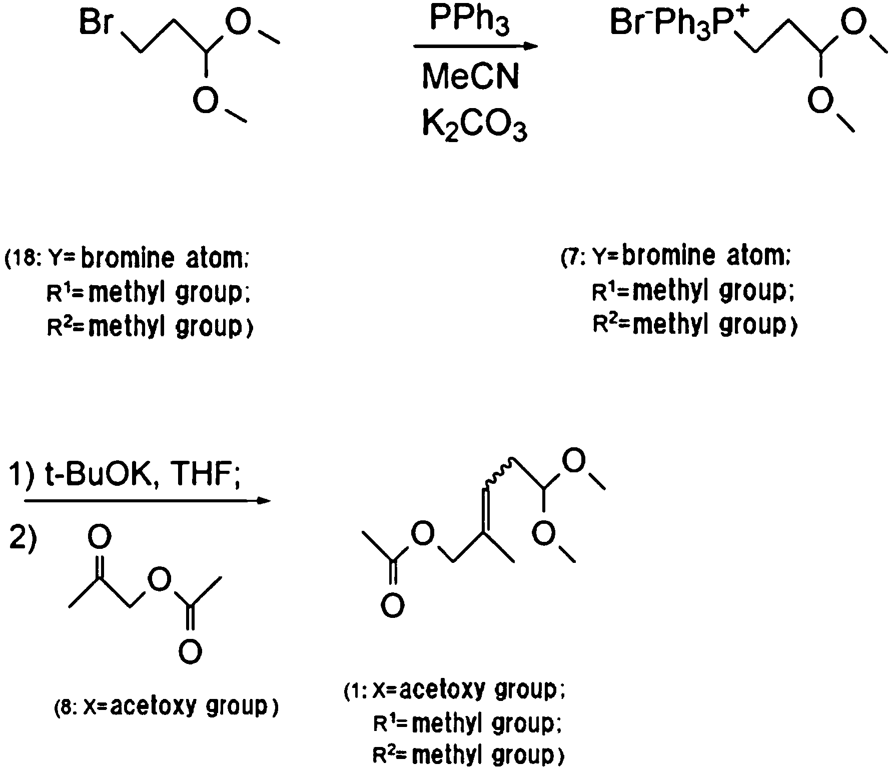

A propyl halide compound having an acetal at 3-position (18: Y = bromine atom; R¹ = methyl group; R² = methyl group) (50 g: 0.273 mol), triphenylphosphine (PPh₃) (65.31 g: 0.249 mol), potassium carbonate (K₂CO₃) (0.97 g: 0.007 mol), and acetonitrile (MeCN) (47.4 g) were placed in a reactor in a nitrogen atmosphere and refluxed with stirring for 12 hours to prepare a propyltriphenylphosphonium halide compound having an acetal at 3-position (7: Y = bromine atom; R¹ = methyl group; R² = methyl group). The internal temperature of the reaction solution was cooled to a room temperature (20 to 25°C), and then tetrahydrofuran (THF) (189.6 g) was added. The internal temperature was then cooled to 0 to 5°C, t-butoxy potassium (t-BuOK) (27.40 g: 0.244 mol) was added, and the reaction mixture was stirred for 1 hour. A 2-propanone compound having a leaving group X (8: X = acetoxy group) (24.73 g: 0.213 mol) prepared as in Synthetic Example 2 was then added dropwise for 33 minutes at an internal temperature of lower than 5°C, and the reaction mixture was stirred for 3 hours. n-Hexane (10 g) was then added dropwise to the reaction solution for 1 minute. An aqueous solution of acetic acid (prepared from purified water (118.5 g) and acetic acid (2.84 g)) was then added to the reaction solution, and the reaction solution was stirred for 30 minutes. After the completion of the stirring, the organic layer was separated. The organic layer thus separated was distilled at a reduced pressure to obtain the target compound, a 4-methyl-3-pentenal acetal compound having an acetoxy group as a leaving group X at 5-position (1: X = acetoxy group; R¹ = methyl group; R² = methyl group) (14.01 g: 0.056 mol). The yield calculated from all fractions including a fore-running fraction was 27.75%.

4-Methyl-3-pentenal acetal compound having leaving group X at 5-position (1: X = acetoxy group; R¹ = methyl group; R² = methyl group):
IR (D-ATR): v = 2940, 2832, 1739, 1449, 1368, 1232, 1194, 1125, 1074, 1057, 1024, 959, 920, 863, 607 cm⁻¹.
¹H-NMR (500 MHz, CDCl₃): 1.76 (3H, s-like), 2.05 (3H, s), 2.37-2.41 (2H, t-like, J = 6.5 Hz), 3.31 (6H, s), 4.34 (1H, t, J = 5.7 Hz), 4.56 (2H, s), 5.39 (1H, t-like, J = 5.4 Hz) ppm.
¹³C-NMR (125 MHz, CDCl₃): δ = 20.85, 21.50, 31.37, 52.97, 63.11, 103.97, 124.36, 132.43, 170.96 ppm.
GC-MS (EI, 70 eV): 31, 43, 58, 75, 97, 111, 127, 141, 157, 170, 201.

### Example 4: Preparation of nucleophilic reagent, 3-butenyl compound (2: R³ = hydrogen atom; M = MgCl), and synthesis of 3-isopropenyl-6-heptenal acetal compound (3: R¹ and R² = ethylene group; R³ = hydrogen atom)

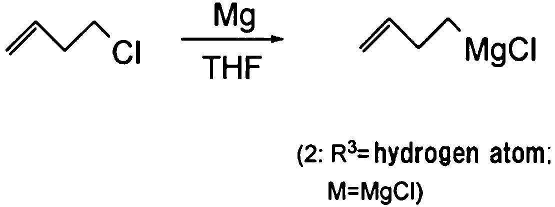

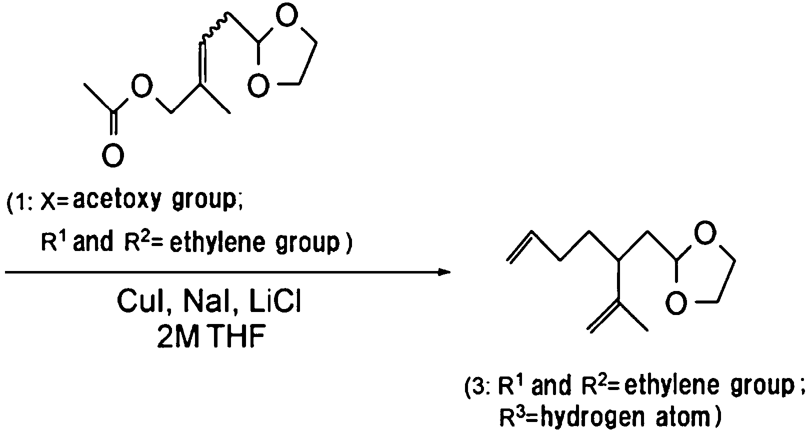

Magnesium (6.32 g: 0.260 mol) and tetrahydrofuran (THF) (24.3 g) were placed in a reactor (in Example 4, referred to as a first reactor) in a nitrogen atmosphere and heated to 65°C, and then stirred for 15 minutes. A mixed solution of 1-chloro-3-butene (22.00 g: 0.243 mol) and tetrahydrofuran (THF) (48.6 g) was then added dropwise to the first reactor for 1 hour, and the reaction mixture was then stirred for 2 hours at an internal temperature of 70 to 80°C to prepare a nucleophilic reagent, 3-butenyl compound (2: R³ = hydrogen atom; M = MgCl). The nucleophilic reagent, 3-butenyl compound (2), thus obtained was then cooled to a room temperature.

Lithium chloride (LiCl) (3.29 g), sodium iodide (NaI) (23.31 g), and 2-methyltetrahydrofuran (2MTHF) (126.36 g) were then placed in a reactor (in Example 4, referred to as a second reactor) other than the aforesaid first reactor in a nitrogen atmosphere and then stirred for 1 hour at a room temperature. Copper(I) iodide (CuI) (3.70 g) was then added to the second reactor, and the reaction mixture was stirred for 1 hour at a room temperature. A 4-methyl-3-pentenal acetal compound having a leaving group X at 5-position (1: X = acetoxy group; R¹ and R² = ethylene group) (10.38 g: 0.049 mol) prepared as in Example 1 was then added to the second reactor, and then the reaction mixture was cooled to 0°C or lower with stirring. The total amount of the nucleophilic reagent, 3-butenyl compound (2), thus prepared was then added dropwise to the second reactor for 5 hours at -5 to 5°C. After the completion of the dropwise addition, the reaction mixture was stirred for 1 hour. A mixture of purified water (22.8 g), ammonium chloride (2.6 g), and hexane (50 g) was then added to the second reactor, and the reaction mixture was stirred for 30 minutes. After the completion of the stirring, the organic layer was separated. The organic layer thus separated was subjected to typical post-treatment of washing and then concentration to obtain a crude product (9.48 g) of a 3-isopropenyl-6-heptenal acetal compound (3: R¹ and R² = ethylene group; R³ = hydrogen atom). The product ratio of the 3-isopropenyl-6-heptenal acetal compound (3) to the 4-methyl-3,8-nonadienal acetal compound (3') was 94:6, and the crude yield was 88.48%.

3-Isopropenyl-6-heptenal acetal compound (3: R¹ and R² = ethylene group; R³ = hydrogen atom):
IR (D-ATR): v = 3074, 2974, 2927, 2881, 2781, 1642, 1475, 1440, 1413, 1376, 1361, 1210, 1141, 1081, 1038, 996, 942, 910, 893, 845, 643, 584 cm⁻¹.
¹H-NMR (500 MHz, CDCl₃): 1.38-1.51 (2H, m), 1.58-1.64 (4H, m), 1.74-1.80 (1H, m), 1.88-2.06 (2H, m), 2.29-2.40 (1H, m), 3.76-4.01 (4H, m)4.75-4.80 (3H, m), 4.90-5.03 (2H, m), 5.74-5.83 (1H, m) ppm.
¹³C-NMR (125 MHz, CDCl₃): δ = 18.00, 31.34, 32.62, 37.67, 42.69, 64.68, 64.71, 103.42, 112.28, 114.38, 138.71, 146.32 ppm.
GC-MS (EI, 70 eV): 29, 45, 55, 73, 86, 99, 113, 123, 134, 143, 153, 168, 181, 195.

### Example 5: Preparation of nucleophilic reagent, 3-butenyl compound (2: R³ = hydrogen atom; M = MgCl), and synthesis of 3-isopropenyl-6-heptenal acetal compound (3: R¹ and R² = ethylene group; R³ = hydrogen atom)

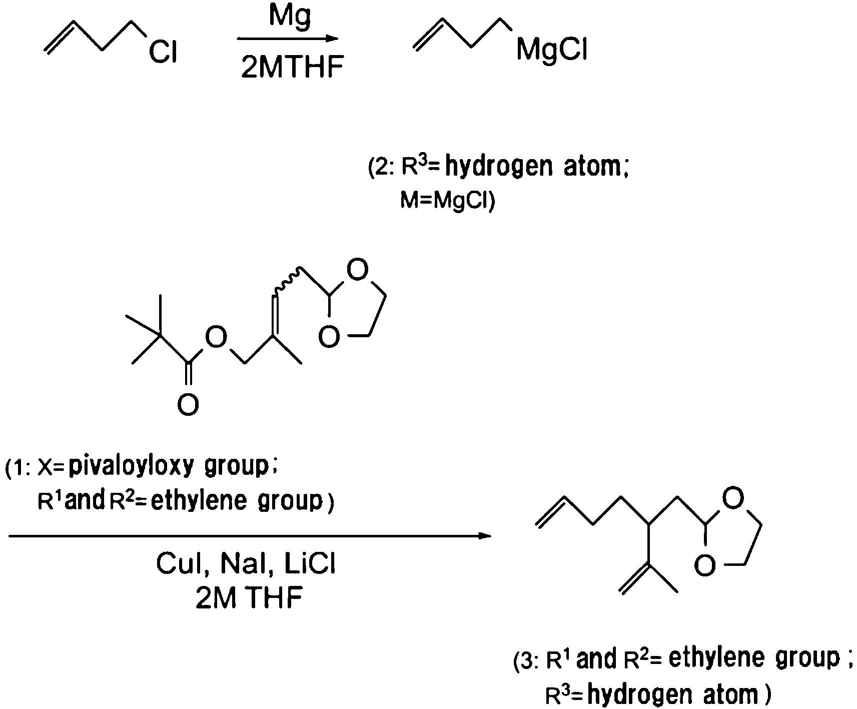

Magnesium (16.77 g: 0.690 mol) and 2-methyltetrahydrofuran (2MTHF) (66.3 g) were placed in a reactor (in Example 5, referred to as a first reactor) in a nitrogen atmosphere, heated to 70°C, and stirred for 20 minutes. A mixed solution of 1-chloro-3-butene (60.03 g: 0.663 mol) and 2-methyltetrahydrofuran (2MTHF) (132.6 g) was then added dropwise to the first reactor for 2 hours, and then the reaction mixture was stirred for 2 hours at an internal temperature of 70 to 80°C to prepare a nucleophilic reagent, 3-butenyl compound (2: R³ = hydrogen atom; M = MgCl). The nucleophilic reagent, 3-butenyl compound (2), thus obtained was then cooled to a room temperature.

Lithium chloride (LiCl) (11.24 g), sodium iodide (NaI) (79.51 g), and 2-methyltetrahydrofuran (2MTHF) (574.60 g) were then placed in a reactor (in Example 5, referred to as a second reactor) other than the aforesaid first reactor in a nitrogen atmosphere and stirred for 1 hour at a room temperature. Copper(I) iodide (CuI) (12.63 g) was then added to the second reactor, and the reaction mixture was stirred for 1 hour at a room temperature. A 4-methyl-3-pentenal acetal compound having a leaving group X at 5-position (1: X = pivaloyloxy group; R¹ and R² = ethylene group) (55.30 g: 0.221 mol) prepared as in Example 2 was then added to the second reactor, and the reaction mixture was cooled to 5°C or lower with stirring. The total amount of the nucleophilic reagent, 3-butenyl compound (2), thus prepared was then added dropwise to the second reactor for 13 hours at 5°C or lower. After the completion of the dropwise addition, the reaction mixture was stirred for 4 hours. A mixture of purified water (311.61 g) and ammonium chloride (35.47 g) was then added to the second reactor, and the reaction mixture was stirred for 30 minutes. After the completion of the stirring, the organic layer was separated. The organic layer thus separated was subjected to typical post-treatment of washing and then concentration to obtain a crude product (46.40 g) of a 3-isopropenyl-6-heptenal acetal compound (3: R¹ and R² = ethylene group; R³ = hydrogen atom). The product ratio of the 3-isopropenyl-6-heptenal acetal compound (3) to the 4-methyl-3,8-nonadienal acetal compound (3') was 98:2, and the crude yield was 98.37%.

### Example 6: Preparation of a nucleophilic reagent, 3-butenyl compound (2: R³= methyl group; M = MgCl), and synthesis of a 3-isopropenyl-6-heptenal acetal compound (3: R¹ and R² = ethylene group; R³ = methyl group)

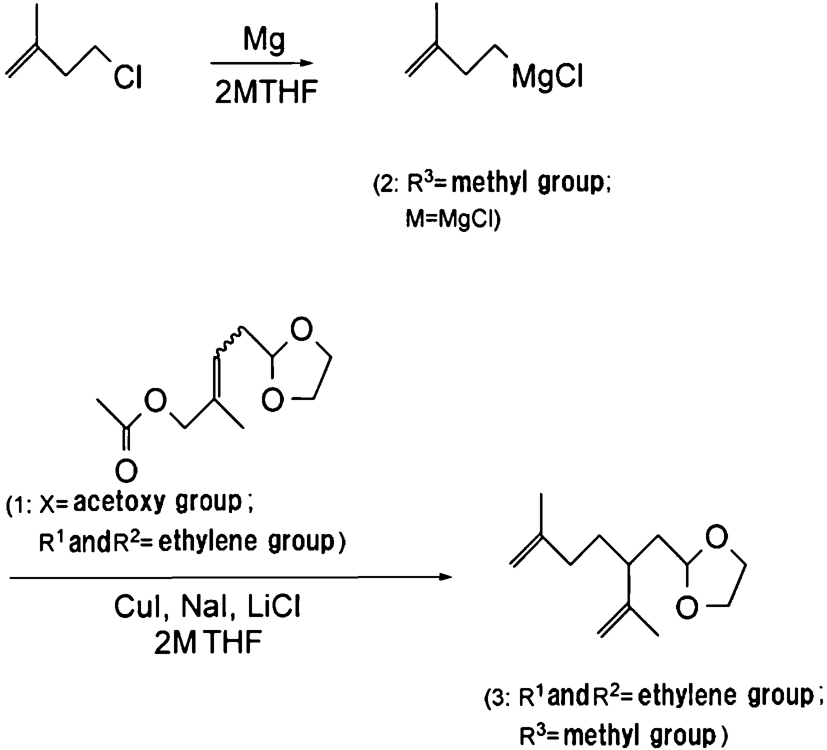

Magnesium (48.65 g: 2.002 mol) and 2-methyltetrahydrofuran (2MTHF) (187 g) were placed in a reactor (in Example 6, referred to as a first reactor) in a nitrogen atmosphere, heated to 70°C, and stirred for 15 minutes. A mixed solution of 1-chloro-3-methyl-3-butene (195.67 g: 1.871 mol) and 2-methyltetrahydrofuran (2MTHF) (374 g) was then added dropwise to the first reactor for 210 minutes, and the reaction mixture was stirred for 2 hours at an internal temperature of 70 to 80°C to prepare a nucleophilic reagent, 3-butenyl compound (2: R³ = methyl group; M = MgCl). The nucleophilic reagent, 3-butenyl compound (2), thus obtained was then cooled to a room temperature.

Lithium chloride (LiCl) (25.39 g), sodium iodide (NaI) (179.52 g) and 2-methyltetrahydrofuran (2MTHF) (1,297.4 g) were then placed in a reactor (in Example 6, referred to as a second reactor) other than the aforesaid first reactor in a nitrogen atmosphere and stirred for 1 hour at a room temperature. Copper(I) iodide (CuI) (28.52 g) was then added to the second reactor, and the reaction mixture was stirred for 1 hour at a room temperature. A 4-methyl-3-pentenal acetal compound having a leaving group X at 5-position (1: X = acetoxy group; R¹ and R² = ethylene group) (100 g: 0.468 mol) prepared as in Example 3 was then added to the second reactor, and the reaction mixture was cooled to 5°C or lower with stirring. The total amount of the nucleophilic reagent, 3-butenyl compound (2), thus prepared was then added dropwise to the second reactor for 19 hours at 5°C or lower. After the completion of the dropwise addition, the reaction mixture was stirred for 1 hour. A mixture of purified water (395 g) and ammonium chloride (26.7 g) was then added to the second reactor, and the reaction mixture was stirred for 30 minutes. After the completion of the stirring, the organic layer was separated. The organic layer thus separated was subjected to typical post-treatment of washing and then concentration to obtain a crude product (105.70 g) of a 3-isopropenyl-6-heptenal acetal compound (3: R¹ and R² = ethylene group; R³ = methyl group). The product ratio of the 3-isopropenyl-6-methyl-6-heptenal acetal compound (3) to the 4,8-dimethyl-3,8-nonadienal acetal compound (3') was 96:4, and the crude yield was 92.09%.

3-Isopropenyl-6-heptenal acetal compound (3: R¹ and R² = ethylene group; R³ = methyl group):
IR (D-ATR): v = 3073, 2967, 2937, 2881, 1786, 1646, 1444, 1411, 1375, 1210, 1139, 1082, 1040, 958, 943, 888, 723, 587, 556 cm⁻¹.
¹H-NMR (500 MHz, CDCl₃): 1.02-1.57 (2H, m), 1.59-1.65 (4H, m), 1.70 (3H, s), 1.73-1.81 (1H, m), 1.85-2.08 (2H, m), 2.26-2.33 (1H, m), 3.78-4.01 (4H, m), 4.64-4.89 (5H, m) ppm.
¹³C-NMR (125 MHz, CDCl₃): δ = 18.04, 22.52, 31.42, 32.29, 37.68, 42.91, 64.68, 64.71, 103.44, 109.65, 112.24, 145.92, 146.43 ppm.
GC-MS (EI, 70 eV): 29, 41, 59, 69, 82, 93, 107, 123, 133, 151, 166, 184.

### Example 7: Synthesis of 3-isopropenyl-6-heptenal compound (4: R³ = hydrogen atom)

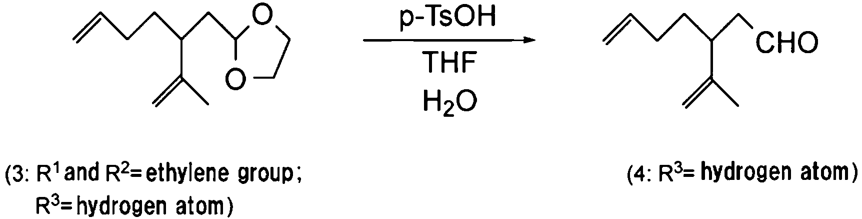

A 3-isopropenyl-6-heptenal acetal compound (3: R¹ and R² = ethylene group; R³ = hydrogen atom) (40 g: 0.187 mol) prepared as in Example 4, p-toluenesulfonic acid (p-TsOH) (17.78 g: 0.0935 mol), tetrahydrofuran (THF) (149.6 g), and purified water (149.6 g) were placed in a reactor in a nitrogen atmosphere, heated to 30 to 35°C, and stirred for 16 hours. Sodium chloride (NaCl) (40 g) was then added, and the reaction mixture was stirred for 30 minutes. After the completion of the stirring, the organic layer was separated. *p*-Toluenesulfonic acid (p-TsOH) (17.78 g: 0.0816 mol) and purified water (149.6 g) were then added to the organic layer thus separated, and the reaction mixture was heated to 30 to 35°C, and then stirred for 16 hours. Sodium chloride (NaCl) (40 g) was then added, and the reaction mixture was stirred for 30 minutes. After the completion of the stirring, the organic layer was separated. The organic layer thus separated was then subjected to typical post-treatment of washing and then concentration to obtain a crude product (30.55 g, quantitative crude yield) of a 3-isopropenyl-6-heptenal compound (4: R³ = hydrogen atom).

3-Isopropenyl-6-heptenal compound (4: R³ = hydrogen atom):
IR (D-ATR): v = 3076, 2975, 2929, 2858, 2720, 1726, 1642, 1441, 1416, 1377, 1185, 996, 897, 640, 549 cm⁻¹.
¹H-NMR (500 MHz, CDCl₃): δ = 1.41-1.54 (2H, m), 1.65 (3H, s-like), 1.92-2.06 (2H, m), 2.37-2.47 (2H, m), 2.66-2.72 (1H, m), 4.77-4.82 (2H, m), 4.93-5.02 (2H, m), 5.73-5.81 (1H, m), 9.65 (1H, t, J = 2.3 Hz) ppm.
¹³C-NMR (125 MHz, CDCl₃): δ = 18.60, 31.10, 32.16, 40.89, 47.36, 112.68, 114.87, 138.07, 145.49, 202.22 ppm.
GC-MS (EI, 70 eV): 27, 41, 55, 69, 81, 95, 108, 123, 137, 151.

### Example 8: Synthesis of 3-isopropenyl-6-heptenal compound (4: R³ = hydrogen atom)

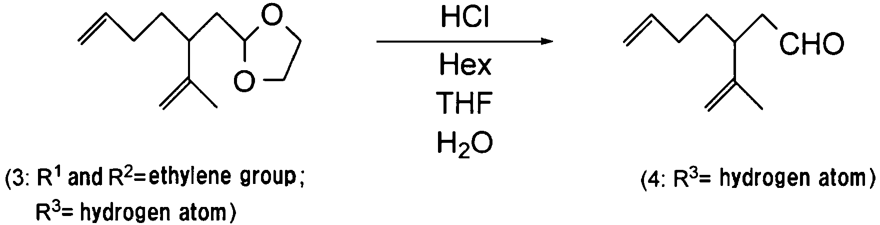

A 3-isopropenyl-6-heptenal acetal compound (3: R¹ and R² = ethylene group; R³ = hydrogen atom) (2 g: 0.00903 mol) prepared as in Example 5, n-hexane (Hex) (7.3 ml), 20% hydrochloric acid (6.0 g), and purified water (4.4 g) were placed in a reactor in a nitrogen atmosphere and stirred for 5 hours at a room temperature. The reaction mixture was then heated to 40°C and stirred for 1.5 hours. After the completion of the stirring, the organic layer was separated. 20% hydrochloric acid (6.0 g) and purified water (4.4 g) were then added to the organic layer thus separated, and the reaction mixture was heated to 40°C and stirred for 10 hours. Tetrahydrofuran (THF) (5 ml) was then added to the reaction solution, and the reaction solution was stirred for 17 hours at 40°C. The organic layer was then separated. 15% hydrochloric acid (10 g) was added to the organic layer thus separated, and the reaction mixture was heated to 40°C and stirred for 3 hours. The organic layer was then separated. 20% hydrochloric acid (10.0 g) was then added to the organic layer thus separated, and the reaction mixture was heated to 40°C and stirred for 3 hours. After the completion of the stirring, the organic layer was separated. The organic layer thus separated was then subjected to typical post-treatment of washing and then concentration to obtain a crude product (1.84 g) of a 3-isopropenyl-6-heptenal compound (4: R³ = hydrogen atom). The crude yield was 37.78%.

### Example 9: Synthesis of 3-isopropenyl-6-heptenal compound (4: R³ = methyl group)

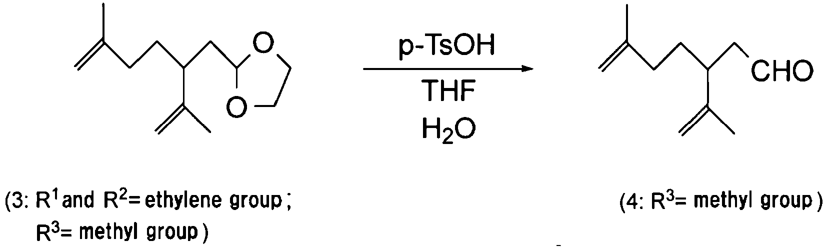

A 3-isopropenyl-6-heptenal acetal compound (3: R¹ and R² = ethylene group; R³ = methyl group) (5 g: 0.0204 mol) prepared as in Example 6, p-toluenesulfonic acid (*p-*TsOH) (0.05 g: 0.000263 mol), tetrahydrofuran (THF) (19 g), and purified water (19 g) were placed in a reactor in a nitrogen atmosphere, heated to 40°C, and stirred for 4.5 hours. *p*-Toluenesulfonic acid (p-TsOH) (0.5 g: 0.00263 mol) was then added to the reaction solution, and the reaction solution was stirred for 8 hours at 40°C. *p-*Toluenesulfonic acid (*p*-TsOH) (1.0 g: 0.00526 mol) was then added to the reaction solution, and the reaction solution was stirred for 10.5 hours at 40°C. p-Toluenesulfonic acid (*p*-TsOH) (1.0 g: 0.00526 mol) was then added to the reaction solution, and the reaction solution was stirred for 5 hours at 40°C. After the completion of the stirring, the organic layer was separated. p-Toluenesulfonic acid (p-TsOH) (2.0 g: 0.0105 mol), tetrahydrofuran (THF) (19 g), and purified water (19 g) were then added to the organic layer thus separated, and the reaction mixture was heated to 40°C and stirred for 9 hours. n-Hexane (30 g) and sodium chloride (NaCl) (10 g) were then added to the reaction solution, and the reaction solution was stirred for 30 minutes. After the completion of the stirring, the organic layer was separated. The organic layer thus separated was then subjected to typical post-treatment of washing and then concentration to obtain a crude product (4.01 g) of a 3-isopropenyl-6-heptenal compound (4: R³ = methyl group). The crude yield was 69.11%.

3-Isopropenyl-6-heptenal compound (4: R³ = methyl group):
IR (D-ATR): v = 3074, 2969, 2936, 2719, 1726, 1647, 1448, 1376, 1070, 1011, 890, 558, 434 cm⁻¹.
¹H-NMR (500 MHz, CDCl₃): 1.47-1.55 (2H, m), 1.66 (3H, s-like), 1.70 (3H, s), 1.89-1.99 (2H, m), 2.38-2.48 (2H, m), 2.66 (1H, quin, J = 7.3 Hz), 4.65-4.71 (2H, m), 4.77-4.82 (2H, m), 9.65 (1H, t-like, J = 2.5 Hz) ppm.
¹³C-NMR (125 MHz, CDCl₃): δ = 18.64, 22.42, 30.89, 35.04, 41.05, 47.41, 110.11, 112.66, 145.26, 145.61, 202.25 ppm.
GC-MS (EI, 70 eV): 29, 41, 55, 69, 81, 97, 107, 122, 137, 151, 165.

### Example 10: Synthesis of 3-isopropenyl-6-heptenal compound (4: R³ = methyl group)

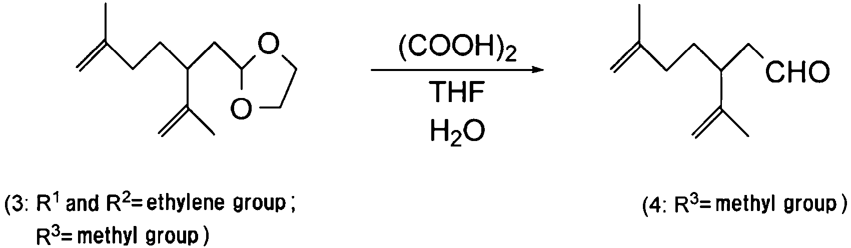

A 3-isopropenyl-6-heptenal acetal compound (3: R¹ and R² = ethylene group; R³ = methyl group) (10 g: 0.0408 mol) prepared as in Example 6, oxalic acid ((COOH)₂) (17.96 g: 0.1425 mol), tetrahydrofuran (THF) (38 g), and purified water (38 g) were placed in a reactor in a nitrogen atmosphere, heated to 40°C, and stirred for 28.5 hours. Sodium chloride (NaCl) (10 g) was then added to the reaction solution, and the reaction solution was stirred for 30 minutes. After the completion of the stirring, the organic layer was separated. Oxalic acid ((COOH)₂) (17.96 g: 0.1425 mol), tetrahydrofuran (THF) (38 g), and purified water (38 g) were added to the organic layer thus separated, and the reaction mixture was heated to 40°C and stirred for 5 hours. n-Hexane (30 ml) and sodium chloride (NaCl) (10 g) were then added, and the reaction mixture was stirred for 30 minutes. After the completion of the stirring, the organic layer was separated. The organic layer thus separated was then subjected to typical post-treatment of washing and then concentration to obtain a crude product (5.38 g) of a 3-isopropenyl-6-heptenal compound (4: R³ = methyl group). The crude yield was 49.05%.

### Example 11: Synthesis of 3-isopropenyl-6-heptenal compound (4: R³ = methyl group)

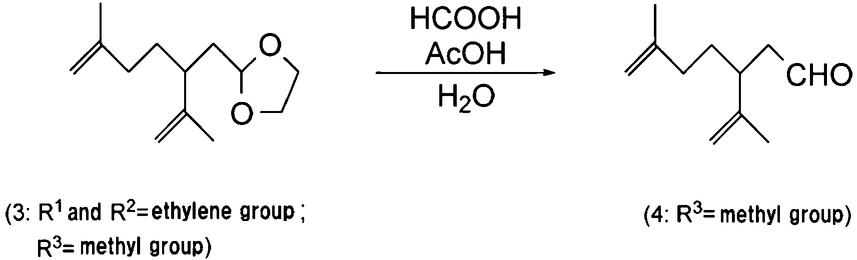

A 3-isopropenyl-6-heptenal acetal compound (3: R¹ and R² = ethylene group; R³ = methyl group) (10 g: 0.0408 mol) prepared as in Example 6, formic acid (2 g), acetic acid (4.91 g), and purified water (3.56 g) were placed in a reactor in a nitrogen atmosphere, heated to 110°C, and stirred for 1 hour. The reaction solution was cooled to a room temperature, then sodium chloride (NaCl) (10 g) was added, and the reaction mixture was stirred for 30 minutes. After the completion of the stirring, the organic layer was separated. Purified water (3.56 g) was added to the organic layer thus separated, and the reaction mixture was heated to 110°C and stirred for 2 hours. The reaction solution was cooled to a room temperature, and then n-hexane (10 g) was added. The reaction mixture was stirred for 30 minutes. After the completion of the stirring, the organic layer was separated. The organic layer thus separated was then subjected to typical post-treatment of washing and then concentration to obtain a crude product (3.31 g) of a 3-isopropenyl-6-heptenal compound (4: R³ = methyl group). The crude yield was 7.35%.

### Example 12: Synthesis of 6-isopropenyl-3-methyl-3,9-decadien-1-ol compound (5: R³ = hydrogen atom)

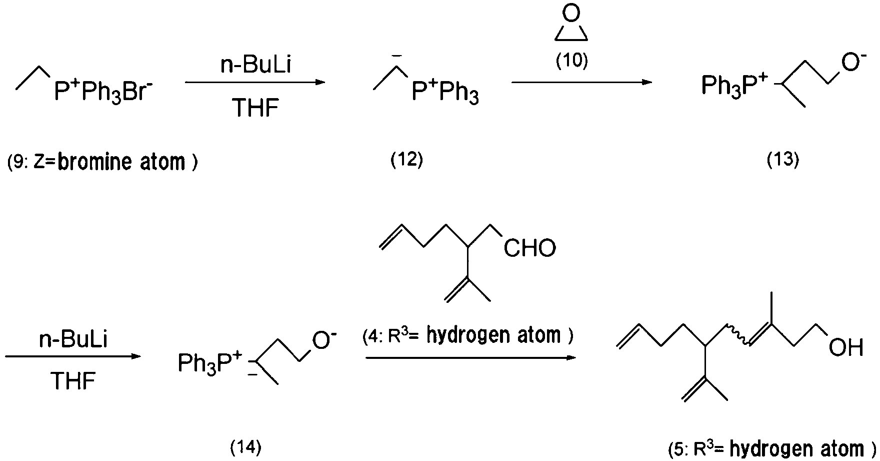

An ethyltriphenylphosphonium halide compound (9: Z = bromine atom) (24.39 g, 0.0788 mol) and tetrahydrofuran (THF) (197.1 g) were placed in a reactor in a nitrogen atmosphere and cooled to 0°C. A solution of n-butyllithium (n-BuLi) (28.14 ml: 0.0788 mol) in hexane was added dropwise to the reaction solution for 20 minutes, and then the reaction mixture was stirred for 15 minutes to prepare ylide (12). Ethylene oxide (10) (3.47 g: 0.0788 mol) was added dropwise to the reaction solution for 5 minutes, and then the reaction mixture was stirred for 1.5 hours at 15 to 20°C to prepare 3-triphenylphosphoniobutoxide (13). The reaction solution was cooled to 0°C, and then a solution of n-butyllithium (n-BuLi) (24.64 ml: 0.0690 mol) in hexane was added dropwise for 12 minutes. The reaction mixture was then stirred for 15 minutes to prepare phosphorus ylide (14). A 3-isopropenyl-6-heptenal compound (4: R³ = hydrogen atom) (10.00 g: 0.0628 mol) prepared as in Example 7 was then added dropwise for 20 minutes, and the reaction mixture was stirred for 1 hour at an internal temperature of 0°C. The internal temperature was then increased to a room temperature, and the reaction mixture was stirred for 3 hours. Purified water (197.1 g) was then added to the reaction solution, and the reaction solution was stirred for 30 minutes. The organic layer was then separated. The organic layer thus separated was then subjected to typical post-treatment of drying and then concentration to obtain a crude product (16.58 g, quantitative crude yield) of the target compound, 6-isopropenyl-3-methyl-3,9-decadien-1-ol compound (5: R³ = hydrogen atom).

6-Isopropenyl-3-methyl-3,9-decadien-1-ol compound (5: R³ = hydrogen atom):
IR (D-ATR): v = 3341, 3074, 2966, 2927, 1642, 1441, 1375, 1185, 1044, 995, 909, 890, 641, 559 cm⁻¹.
¹H-NMR (500 MHz, CDCl₃): δ = 1.31-1.49 (2H, m), 1.59-1.71 (7H, m), 1.88-2.16 (5H, m), 2.21-2.35 (2H, m), 3.59-3.67 (2H, m), 4.66-4.77 (2H, m), 4.91-5.01 (2H, m), 5.14-5.27 (1H, m), 5.75-5.83 (1H, m) ppm.
¹³C-NMR (125 MHz, CDCl₃): δ = 15.76, 17.98, 18.44, 23.35, 31.58, 32.00, 32.09, 32.11, 35.16, 42.60, 47.14, 47.15, 59.60, 60.52, 111.70, 111.85, 114.27, 114.35, 126.80, 126.98, 131.46, 131.48, 138.83, 138.92, 147.13, 147.42 ppm.
GC-MS (EI, 70 eV): 29, 41, 55, 67, 81, 93, 107, 121, 135, 149, 163, 177, 193, 208.

### Example 13: Synthesis of 6-isopropenyl-3-methyl-3,9-decadienyl carboxylate compound (6: R³ = hydrogen atom; R⁴ = methyl group)

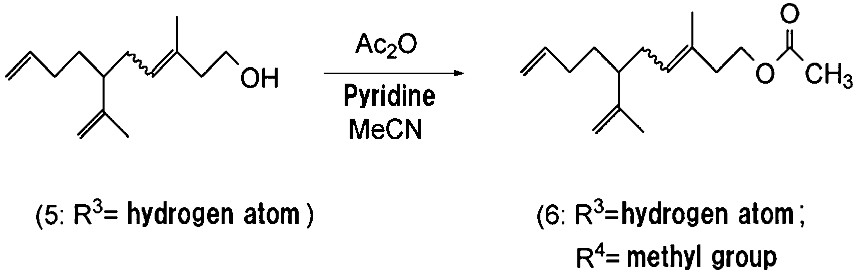

A 6-isopropenyl-3-methyl-3,9-decadien-1-ol compound (5: R³ = hydrogen atom) (15.88 g) prepared as in Example 12, acetic anhydride (Ac₂O) (12.94 g: 0.106 mol), pyridine (21.04 g: 0.266 mol), and acetonitrile (MeCN) (15.88 g) were placed in a reactor in a nitrogen atmosphere and stirred for 3 hours at a room temperature. Purified water (39.52 g) and n-hexane (9.88 g) were added to the reaction solution, and the reaction solution was stirred for 30 minutes. The organic layer was then separated. The organic layer thus separated was then subjected to typical washing, drying, concentration, and distillation to obtain the target compound, 6-isopropenyl-3-methyl-3,9-decadienyl carboxylate compound (6: R³ = hydrogen atom; R⁴ = methyl group) (7.69 g: 0.0307 mol). The two-step yield calculated from all fractions including a fore-running fraction was 63.21%.

### 6-Isopropenyl-3-methyl-3,9-decadienyl carboxylate compound (6: R³ = hydrogen atom; R⁴ = methyl group)

IR (D-ATR): v = 3074, 2968, 2926, 1742, 1642, 1442, 1364, 1237, 1041, 995, 909, 890 cm⁻¹.

¹H-NMR (500 MHz, CDCl₃): δ = 1.36-1.50 (2H, m), 1.60-1.72 (6H, m), 1.88-2.10 (8H, m), 2.21-2.36 (2H, m), 4.07-4.13 (2H, m), 4.66-4.77 (2H, m), 4.91-5.01 (2H, m), 5.12-5.21 (1H, m), 5.75-5.83 (1H, m) ppm.

¹³C-NMR (125 MHz, CDCl₃): δ = 16.22, 18.43, 20.99, 21.03, 23.57, 31.24, 31.59, 31.90, 31.97, 32.13, 38.61, 46.87, 47.04, 62.69, 63.05, 111.53, 111.67, 114.26, 114.28, 125.69, 126.41, 131.13, 131.25, 138.93, 138.96, 147.08, 147.18, 171.07, 171.11 ppm.

GC-MS (EI, 70 eV): 29, 43, 55, 67, 81, 93, 107, 121, 135, 147, 161, 175, 190, 207, 222, 235, 250.

## Claims

1. A process for preparing a 3-isopropenyl-6-heptenal compound of the following general formula (4): 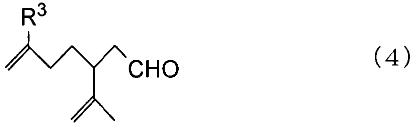 wherein R³ represents a hydrogen atom or a methyl group,
the process comprising at least steps of:
subjecting a 4-methyl-3-pentenal acetal compound of the following general formula (1), having a leaving group X at 5-position: 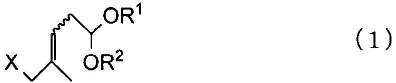
wherein R¹ and R² represent, independently of each other, a monovalent hydrocarbon group having 1 to 10 carbon atoms; or R¹ and R² are bonded to each other and represent a divalent hydrocarbon group having 2 to 10 carbon atoms, X represents an acyloxy group having 1 to 10 carbon atoms including the carbon of the carbonyl group, an alkanesulfonyloxy group having 1 to 10 carbon atoms, an arenesulfonyloxy group having 6 to 20 carbon atoms, or a halogen atom, and the wavy line represents the E-form, the Z-form, or a mixture thereof,
to a nucleophilic substitution reaction with a nucleophilic reagent, 3-butenyl compound, of the following general formula (2): 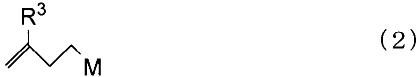
wherein R³ is as defined above, and M represents Li, MgW¹, ZnW¹, Cu, CuW¹, or CuLiW¹, and W¹ represents a halogen atom or a CH₂=C(R³)CH₂CH₂- group,
to obtain a 3-isopropenyl-6-heptenal acetal compound of the following general formula (3): 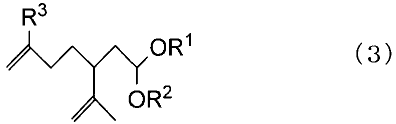
wherein R¹, R², and R³ are as defined above, and
subjecting the 3-isopropenyl-6-heptenal acetal compound (3) thus obtained to an acid hydrolysis reaction to obtain the aforesaid 3-isopropenyl-6-heptenal compound (4).

2. A process for preparing a 6-isopropenyl-3-methyl-3,9-decadienyl carboxylate compound of the following general formula (6): 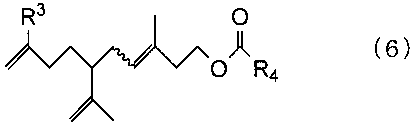
wherein R³ represents a hydrogen atom or a methyl group, R⁴ represents a hydrogen atom or a monovalent hydrocarbon group having 1 to 10 carbon atoms, and the wavy line represents the E-form, the Z-form, or a mixture thereof,
the process comprising at least steps of:
the aforesaid process, according to claim 1, for preparing the 3-isopropenyl-6-heptenal compound (4),
subjecting the 3-isopropenyl-6-heptenal compound (4) thus obtained to a four-carbon homologation reaction including a Wittig reaction with an ethyltriphenylphosphonium halide compound and ethylene oxide to obtain a 6-isopropenyl-3-methyl-3,9-decadien-1-ol compound of the following general formula (5): 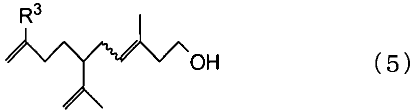
wherein R³ and the wavy line are as defined above, and
subjecting the 6-isopropenyl-3-methyl-3,9-decadien-1-ol compound (5) thus obtained to an esterification reaction to obtain the aforesaid 6-isopropenyl-3-methyl-3,9-decadienyl carboxylate compound (6).

3. A process for preparing a 4-methyl-3-pentenal acetal compound of the following general formula (1), having a leaving group X at 5-position: 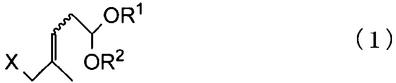 wherein R¹ and R² represent, independently of each other, a monovalent hydrocarbon group having 1 to 10 carbon atoms; or R¹ and R² are bonded to each other and represent a divalent hydrocarbon group having 2 to 10 carbon atoms, X represents an acyloxy group having 1 to 10 carbon atoms including the carbon of the carbonyl group, an alkanesulfonyloxy group having 1 to 10 carbon atoms, an arenesulfonyloxy group having 6 to 20 carbon atoms, or a halogen atom, and the wavy line represents the E-form, the Z-form, or a mixture thereof,
the process comprising:
subjecting a propyltriphenylphosphonium halide compound of the following general formula (7), having an acetal at 3-position: 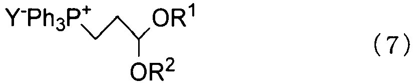
wherein R¹ and R² represent, independently of each other, a monovalent hydrocarbon group having 1 to 10 carbon atoms; or R¹ and R² are bonded to each other and represent a divalent hydrocarbon group having 2 to 10 carbon atoms, Y represents a halogen atom, and Ph represents a phenyl group,
to a Wittig reaction with a 2-propanone compound of the following general formula (8), having a leaving group X: 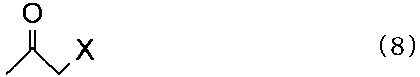
wherein X is as defined above,
to obtain the aforesaid 4-methyl-3-pentenal acetal compound (1).

4. A process for preparing a 3-isopropenyl-6-heptenal compound of the following general formula (4): 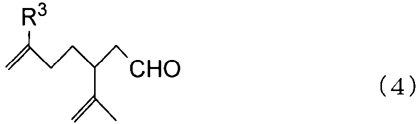
wherein R³ represents a hydrogen atom or a methyl group ,
the process comprising at least steps of:
the aforesaid process, according to claim 3, for preparing the 4-methyl-3-pentenal acetal compound (1) having a leaving group X at 5-position,
subjecting the aforesaid 4-methyl-3-pentenal acetal compound (1) having a leaving group X at 5-position to a nucleophilic substitution reaction with a nucleophilic reagent, 3-butenyl compound, of the following general formula (2): 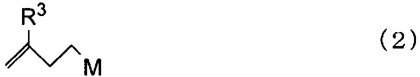
wherein R³ is as defined above, and M represents Li, MgW¹, ZnW¹, Cu, CuW¹, or CuLiW¹, and W¹ represents a halogen atom or a CH₂=C(R³)CH₂CH₂- group,
to obtain a 3-isopropenyl-6-heptenal acetal compound of the following general formula (3): 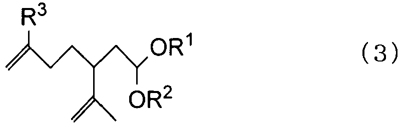
wherein R¹ and R² represent, independently of each other, a monovalent hydrocarbon group having 1 to 10 carbon atoms; or R¹ and R² are bonded to each other and represent a divalent hydrocarbon group having 2 to 10 carbon atoms, X represents an acyloxy group having 1 to 10 carbon atoms including the carbon of the carbonyl group, an alkanesulfonyloxy group having 1 to 10 carbon atoms, an arenesulfonyloxy group having 6 to 20 carbon atoms, or a halogen atom, and R³ are as defined above, and
subjecting the 3-isopropenyl-6-heptenal acetal compound (3) thus obtained to an acid hydrolysis reaction to obtain the aforesaid 3-isopropenyl-6-heptenal compound (4).

5. A 4-methyl-3-pentenal acetal compound of the following general formula (1), having a leaving group X at 5-position: 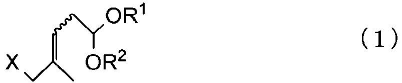 wherein R¹ and R² represent, independently of each other, a monovalent hydrocarbon group having 1 to 10 carbon atoms; or R¹ and R² are bonded to each other and represent a divalent hydrocarbon group having 2 to 10 carbon atoms, X represents an acyloxy group having 1 to 10 carbon atoms including the carbon of the carbonyl group, an alkanesulfonyloxy group having 1 to 10 carbon atoms, an arenesulfonyloxy group having 6 to 20 carbon atoms, or a halogen atom, and the wavy line represents the E-form, the Z-form, or a mixture thereof.
